# EUROPÄISCHE PATENTSCHRIFT

(11) **EP 1 549 631 B1**
(45) Veröffentlichungstag und Bekanntmachung des Hinweises auf die Patenterteilung: **08.01.2014**
(21) Anmeldenummer: 03769344.7
(22) Anmeldetag: 30.09.2003
(51) Int. Cl.: C07D 317/38, C11D 1/83, C11D 1/66

(54) **Alkylglycidolcarbonate als Co-Tenside**
Alkyl glycidol carbonates as co-surfactants
Carbonates d'alkylglycidol en tant qu'agents co-tensioactifs

(30) Priorität: 01.10.2002 DE 10246139
(43) Veröffentlichungstag der Anmeldung: 06.07.2005
(73) Patentinhaber: BASF SE, 67056 Ludwigshafen (DE)
(72) Erfinder: NÖRENBERG, Ralf, 55218 Ingelheim (DE); KLUGE, Michael, 67071 Ludwigshafen (DE); WULFF, Christian, 68163 Mannheim (DE); TROPSCH, Jürgen, 67354 Römerberg (DE); SCHOLTISSEK, Martin, 67157 Wachenheim (DE)
(86) Internationale Anmeldenummer: PCT/EP2003/010841
(87) Internationale Veröffentlichungsnummer: WO 2004/031167

(56) Entgegenhaltungen:
- EP-A- 1 070 733
- EP-A- 1 116 751
- EP-A1- 0 599 265
- WO-A-01/30930
- WO-A-01/52340
- WO-A-97/04059
- WO-A-97/19159
- WO-A-98/00418
- WO-A1-99/66016
- DE-A- 1 769 119
- DE-A- 2 159 991
- DE-A- 2 344 197
- US-A- 4 341 905
- US-B1- 6 447 952
- PATENT ABSTRACTS OF JAPAN vol. 011, no. 332 (C-455), 29. Oktober 1987 (1987-10-29) & JP 62 114985 A (NEOS CO LTD), 26. Mai 1987 (1987-05-26)
- CALO V. ET AL: "Cyclic Carbonate Formation from Carbon Dioxide and Oxiranes in Tetrabutylammonium Halides as Solvents and Catalysts", ORGANIC LETTERS, vol. 4, no. 15, 2002, pages 2561-2563,

## Beschreibung

Die vorliegende Erfindung bezieht sich auf Alkylglycidolcarbonate und deren Verwendung als Co-Tenside.

Tenside sind sogenannte amphiphile Moleküle, die in ihrer Molekülstruktur einen hydrophoben und einen hydrophilen Anteil aufweisen. Durch diese Eigenschaft können Tenside Grenzflächenfilme und sogenannte Micellen ausbilden. Dabei handelt es sich um Aggregate aus Tensiden, die sich in wässrigen Lösungen bilden und unterschiedliche Gestalt (Kugeln, Stäbchen, Scheiben) annehmen können. Micellen bilden sich oberhalb einer bestimmten Konzentration, der sogenannten kritischen Micell-Bildungskonzentration (KMK). Weiterhin besitzen amphiphile Moleküle die Eigenschaft, Grenzflächenfilme zwischen hydrophoben und hydrophilen Phasen auszubilden und so beispielsweise emulgierend oder schäumend zu wirken.

Co-Tenside haben ebenfalls amphiphile Eigenschaften, die jedoch nicht ausreichen, um alleine Micellen und Grenzflächenfilme bilden zu können. Sie werden jedoch zwischen den Tensiden eingelagert und bewirken eine Steigerung der Packungsdichte der Amphiphilen (Tenside und Co-Tenside) in den von diesen geformten Gebilden wie Micellen oder Grenzflächen. Dadurch erniedrigt sich neben der kritischen Micellbildungskonzentration und der Oberflächenspannung auch die Grenzflächenspannung zwischen der wässrigen Tensidlösung und unpolaren Substanzen wie beispielsweise Ölen, so dass die Aufnahmekapazität des Tensidsystems für diese Stoffe bis hin zur Bildung von Mikroemulsionen steigt. Es resultieren ein höheres Solubilisier- und Emulgiervermögen, eine höhere Reinigungskapazität sowie eine erhöhte Stabilität der Emulsionen und Schäume. Bei Einsatz von Co-Tensiden können Micellen bereits bei deutlich niedrigerer Tensid-Konzentration ausgebildet werden.

Weitere Effekte, die durch den Einsatz der Co-Tenside und die dadurch verstärkte Aggregationstendenz der Amphiphile hervorgerufen werden, sind bekannt. Dies ist zum einen die Aggregationstransformation von sphärischen zu anisometrischen micellaren Assoziaten. Diese Strukturänderung der Micellen hat Auswirkungen auf die Rheologie der die Micellen enthaltenen Lösungen, insbesondere in verdünnten Lösungen. Gleichzeitig tritt im Phasendiagramm eine Verschiebung vorliegender flüssigkristalliner Strukturen zu niedrigeren Konzentrationen auf, wodurch eine bevorzugte Bildung von Gelphasen mit hoher Packungsdichte zu beobachten ist. Als Folge davon treten bereits bei Konzentrationen von deutlich < 10 Gew.-% lamellare Micellstrukturen auf, die sonst erst bei wesentlich höheren Konzentrationen beobachtet werden. Ein weiteres interessantes Phänomen ist das Ausbilden, neben den bekannten flüssigkristallinen Gelphasen, von neuen Überstrukturen, die interessante anwendungstechnische Eigenschaften aufweisen. Von besonderem Interesse sind hierbei vesikuläre Phasen sowie sogenannte L₃-Phasen, die schwammartig ausgebildet sind und mikroemulsionsähnliche Eigenschaften aufweisen. Sie können in verdünnten Konzentrationsbereichen zur Einstellung der Viskosität genutzt werden.

Aus dem Stand der Technik sind eine Anzahl von Verbindungen bzw. Verbindungsklassen bekannt, die als Co-Tenside geeignet sind.

C₅-C₁₀-Alkohole zeigen vorteilhafte Eigenschaften, kommen jedoch häufig wegen ihres charakteristischen Geruchs nicht zum Einsatz.

Niedrig ethoxilierte Alkohole wie beispielsweise niedrig ethoxilierte Laurylalkoholethoxilate, Diethylenglykolmonohexylether oder Propylenglykolbutylether können in einigen Tensidsystemen zu verbesserter Emulgierleistung oder Schaumstabilität führen, weisen aber für Tensidformulierungen mit hohem Aniontensidgehalt eine zu geringe Polarität der Kopfgruppe auf.

Fettsäureethanolamine werden beispielsweise zur Einstellung der Viskosität in Shampoos eingesetzt. Sie stehen jedoch im Verdacht, Nitrosamine auszubilden.

G. J. Smith beschreibt in Seifen, Ölen, Fette, Wachse, 105 (1979, Seiten 319 ff und 345 ff) den Einsatz von Alkylaminoxiden als Co-Tensid in verschiedenen Anwendungen. Auch sie stehen im Verdacht Nitrosamine zu enthalten. Durch eine ausgefüllte, aufwendige Herstellungstechnologie kann das weitgehend vermieden werden.

Analog den Aminoxiden können auch andere zwitterinonische Tenside, wie z. B. Sulfo- oder Carboxylammonio-betaine als Co-Tensid verwendet werden. Bei diesen Produkten ist die Ausbildung von Gelphasen sehr schwach ausgeprägt. Statt dessen haben sie aber den Anwendungsvorteil, dass die Hautreizwirkung von entsprechenden Tensidmischungen abgesenkt wird.

Die WO 98/00418 offenbart Alkylencarbonate, die mit Alkylgruppen substituiert sind, und deren Einsatz als Co-Tenside.

WO 97/04059 betrifft Reinigungsmittelzusammensetzungen, die einen analephotropischen negativ geladenen Komplex enthalten, der aus mindestens einem anionischen Tensid und einem damit komplexierten Alkylencarbonat aufgebaut ist Zusätzlich können die Reinigungsmittelzusammensetzungen gegebenenfalls ein Co-Tensid, einen wasserunlöslichen Kohlenwasserstoff, ein Parfum, eine Lewis-Base oder ein neutrales Polymer enthalten. Das Alkylencarbonat weist einen C₄- bis C₁₄-Alkylrest auf.

Bei den bis jetzt bekannten Anwendungen schwankt das eingesetzte Verhältnis von Co-Tensiden zu Tensiden je nach Anwendung von ca. 1 : 20 bis 1 : 2. In einigen Fällen, wie z. B. Alkylaminoxiden, kann das Co-Tensid auch höher konzentriert sein.

EP-A 1 116 751 betrifft Polycarbonatzusammensetzungen, die eine hervorragende thermische Stabilität aufweisen und deren Verwendung als optische Aufnahmemedien. Die Polycarbonatzusammensetzung enthält ein aromatisches Polycarbonat (Komponente A), eine cyclische Carbonatverbindung, einen Monoester eines bivalenten aliphatischen Alkohols und einer aliphatischen Monocarbonsäure, oder einen Ether, der durch Umsetzung mindestens einer Hydroxylgruppe eines polyvalenten aliphatischen Alkohols in eine aliphatische Oxigruppe erhalten wird (Komponente B), oder einen Halbester eines aliphatischen Alkohols und einer aliphatischen Monocarbonsäure, ein cyclisches Phenol oder eine Verbindung ausgewählt aus phosphoriger Säure, Borsäure und Aminsalzen und Ammoniusalzen davon (Komponente C). Als cyclische Carbonatverbindungen werden Verbindungen der folgenden Formel genannt, 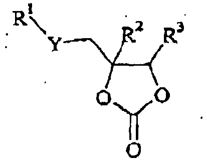 worin R¹ ein Kohlenwasserstoffrest mit 1 bis 20 Kohlenstoffatomen ist und Y eine Bindung, O, COO, oder OC(O)O bedeutet Als Beispielverbindungen werden Verbindungen genannt, worin R¹-YC₁₂H₅-O, C₁₇H₃₅-O, C₁₈H₃₇-O bedeuten Gemäß der Beschreibung handelt es sich bei dem Rest R¹ bevorzugt um einen linearen C₁₀- bis C₂₀-Alkylrest.

US 4,341,905 betrifft Hydroxyalkylphenylether oder. -thioether, die durch Reaktion von cyclischen organischen Carbonaten mit Phenolen oder Thiophenolen in Anwesenheit eines Alkalimetallhalogenidkatalysators erhalten werden. Als cyclische organische Carbonate werden Carbonate der folgenden Formel aufgeführt

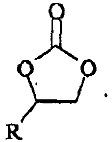

Darin bedeutet R C₁₋₂₀-Alkoxy, Alkoxyalkylen oder (poly)alkoxyalkylen. Bevorzugt ist R CH₂-O-CH(CH₃)₂, CH₂-O-C(CH₃)₃ oder CH₂-O-Ph.

WO 01/52340 betrifft neuartige Elektrolyten und eine Lithiumionenbatterie, worin diese Elektrolyten verwendet werden. Der Elektrolyt enthält ein cyclisches Carbonat der Formel 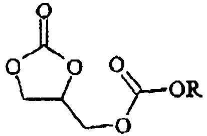 worin R Methyl oder Ethyl bedeutet. Die Herstellung dieser Verbindung erfolgt über die Herstellung von 4-Allyloxymethyl-1,3-dioxolan-2-on.

US 6,447,952 betrifft Alkaliionen leitende Polymerelektrolyte, die Polysiloxane aufweisen, die mit cyclischen Carbonatresten substituiert sind. Zur Herstellung der mit Cyclocarbonatresten substituierten Polysiloxane werden die folgenden Verbindungen eingesetzt:

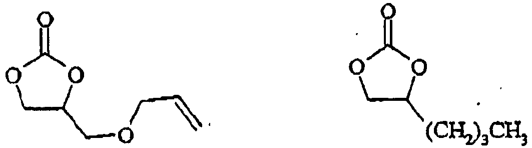

EP-A 1 070 733 betrifft ein Verfahren zur Herstellung von Polyamino-funktionellen Hydroxyurethanoligomeren und Polymeren, die daraus hergestellt werden. Als Ausgangssubstanzen werden unter anderem Verbindungen der folgenden Formeln eingesetzt

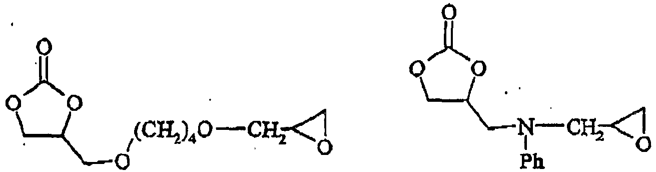

DE-A 2 159 991 betriff Silicium-haltige Dioxolanderivate und ihre Verwendung zur Herstellung von Epoxygruppen enthaltenden Organosilanestem. Die Dioxolanderivate weisen die folgende Formel auf

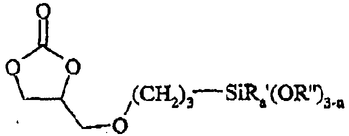

Darin bedeuten a 0 oder 1, R O oder 2 Wasserstoffatome und R' und R" Alkyl. Die Herstellung der vorstehend genannten Dioxolanderivate erfolgt durch Umsetzung von 2-Oxo-4-allyloxymethyl-1,3-dioxolan.

DE-A 23 44 197 betrifft funktionelle Organophosphorsäureester als konservierende Haftvermittler oder Überzüge für Metalle. Als Phosphorsäureester sind unter anderem Verbindungen der folgenden Formel genannt

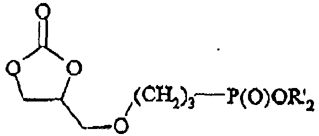

DE-A 1 769 119 betrifft thermoplastische Polyester enthaltende Formmassen. Diese Formmassen enthalten unter anderem cyclische Carbonate der folgenden Formel

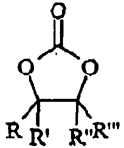

Darin bedeuten R, R', R", R"' Wasserstoff, Alkyl gegebenenfalls enthaltend Sauerstoff, Cycloalkyl, Aralkyl oder Aryl, wobei einer der vorstehend genannten Reste wenigstens eine cyclische Carbonatgruppe enthält.

WO 01/30930 betrifft Silikonverbindungen, die zum Beispiel die folgende Formel aufweisen können.

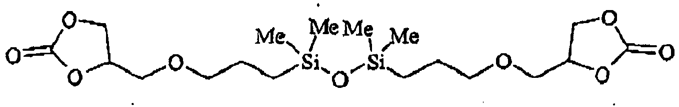

JP-A 62 11 4985 betrifft ein Verfahren zur Herstellung von substituierten 1,3-Dioxolan-2-on-Derivaten der folgenden Formel

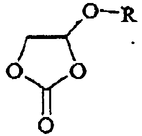

Diese Derivate werden durch Umsetzung von Glycidol mit einem C₁₋₄-Alkohol in Anwesenheit eines basischen Katalysators hergestellt. Somit ist R in der vorstehend genannten Formel eine C₁₋₄-Alkylrest.

EP-A 0599 265 betrifft Polymerverbindungen mit antibakteriellen und antifungalen Eigenschaften sowie Monomere, die als Zwischenprodukte bei der Synthese der Polymerverbindungen dienen und selbst antibakterielle und antifungale Eigenschaften besitzen. In Beispiel 62 wird als Ausgangsprodukt 3-Isobutyroxy-1,2-propylencarbonat eingesetzt.

V. Calo et al., Organic Letters, Bd. 4, Nr. 15, 2002, Seiten 2561-2563 betrifft die Herstellung von cyclischen Carbonaten, ausgehend von Kohlendioxid und Oxiranen in Tetrabutylammoniumhalogeniden als Lösungsmittel und Katalysatoren. Gemäß V. Calo et al, werden unter anderem cyclische Carbonate der Formel 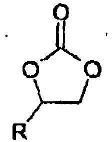 offenbart, worin 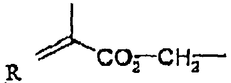 bedeutet.

WO 99/66016 A betrifft die Verbesserung der Löslichkeit von tensidhaltigen Wasch- und Reinigungsmitteln durch Verwendung von unsubstituierten oder in 4- bzw. 4- und 5-Stellung substituierten 1,3-Dioxolan-2-onen als Löslichkeitsverbesserer. Die substituierten Dioxolanone weisen bevorzugt die folgende Formel auf: 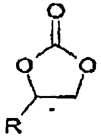 wobei R ein substituierter oder unsubstituierter Alkyl-. Alkenyl- oder Alkylarylrest ist. Besonders bevorzugte Dioxolanone sind Ethylencarbonat und Glycerincarbonat.

Der vorliegenden Bindung liegt die Aufgabe zugrunde, als Co-Tenside geeignete Verbindungen zur Verfügung zu stellen, die die genannten Nachteile nicht aufweisen, insbesondere eine sehr gute Kosteneffizienz und Effektivität zeigen sowie umweltverträglich und frei von Risiken für den Menschen sind.

Diese Aufgabe wird gelöst durch Alkylglycidolcarbonate der allgemeinen Formel I 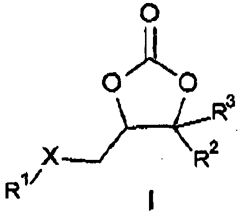 in denen die Symbole X, R¹, R² und R³ die folgende Bedeutung aufweisen:
R¹ ist eine lineare oder verzweigte, unsubstituierte C₃-C₂₉-Alkylgruppe oder eine lineare oder verzweigte, unsubstituierte C₃-C₂₉-Alkenylgruppe, wobei der Substituent R¹ einen mittleren Verzweigungsgrad, der definiert ist als (Anzahl der Methylgruppen pro Molekül)-1, von 0,2 bis 1,6 aufweist, bevorzugt ist R¹ eine lineare oder verzweigte C₃-C₂₁-Alkylgruppe oder eine lineare oder verzweigte C₃-C₂₁-Alkenylgruppe, besonders bevorzugt eine lineare oder verzweigte C₅-C₁₈-Alkylgruppe oder eine lineare oder verzweigte C₃-C₁₈-Alkenylgruppe, ganz besonders bevorzugt eine lineare oder verzweigte C₅-C₁₅-Alkylgruppe oder eine lineare oder verzweigte C₅-C₁₅-Alkenylgruppe;
R² und R³ sind unabhängig voneinander Wasserstoff oder eine lineare oder verzweigte Alkylgruppe, bevorzugt Wasserstoff oder eine lineare oder verzweigte Alkylgruppe mit 1 bis 5 Kohlenstoffatomen, besonders bevorzugt ist mindestens einer der Reste R² oder R³ Wasserstoff, ganz besonders bevorzugt sind Verbindungen der Formel I worin R² und R³ Wasserstoff bedeuten;
X ist ausgewählt aus der Gruppe bestehend aus O und O(CH₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₒ von der allgemeinen Formel I umfasst sind, worin n unterschiedliche Zahlenwerte aufweise bevorzugt ist X O, O(CH₂CHR⁴O)ₙ oder NR⁵, wobei die Bedeutungen für R⁴, R⁵ und n den vorstehend genannten Bedeutungen entsprechen; besonders bevorzugt ist X O,
wobei Alkylglycidolcarbonate der Formel 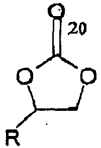 , mit R = CH₂-O-CH(CH₃)₂ und mit R = CH₂-O-CH₂CH(CH₃)
ausgenommen sind (Ausführungsform I).

In einer weiteren Ausführungsform wird die Aufgabe gelöst durch Alkylglycidolcarbonate der allgemeinen Formel I 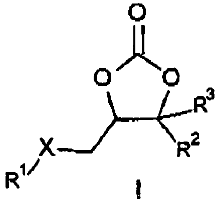 worin die Symbole X, R¹, R² und R³ die folgende Bedeutung aufweisen:
R¹ ist eine lineare oder verzweigte, unsubstituierte C₃-C₂₉-Alkylgruppe oder eine lineare oder verzweigte, unsubstituierte C₃-C₂₉-Alkenylgruppe, bevorzugte eine lineare oder verzweigte C₃-C₁₈-Alkylgruppe oder eine lineare oder verzweigte C₃-C₁₈-Alkenylgruppe, besonders bevorzugt eine lineare oder verzweigte C₅-C₁₈-Alkylgruppe oder eine lineare oder verzweigte C₅-C₁₈-Alkenylgruppe;
R² und R³ sind unabhängig voneinander Wasserstoff oder eine lineare oder verzweigte Alkylgruppe, bevorzugt unabhängig voneinander Wasserstoff oder eine lineare oder verzweigte Alkylgruppe mit 1 bis 5 Kolalenstoffatomen, besonders bevorzugt ist mindestens einer der Reste R² oder R³ Wasserstoff, ganz besonders bevorzugt bedeuten R² und R³ Wasserstoff;
X ist O(CH-₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel 1 umfasst sind, worin n unterschiedliche Zahlenwerte aufweist.

Bevorzugt weist der Substituent R¹ einen mittleren Verzweigungsgrad, der definiert ist als (Anzahl der Methylgruppen pro Molekül)-1, von 0 bis 2,5, vorzugsweise 0,2 bis 1,6, auf (Ausführungsform II).

Die Verbindungen der Formel I eignen sich hervorragend für den Einsatz als Co-Tenside in den üblichen, dem Fachmann bekannten Wasch- und Reinigungsformulierungen.

### a) Phosgen-Weg

Zum einen ist eine Umsetzung von mit einer R¹-X-CH₂-Gruppe funktionalisierten 1,2-Diolen der allgemeinen Formel II mit Phosgen gemäß dem folgenden Reaktionsschema möglich.

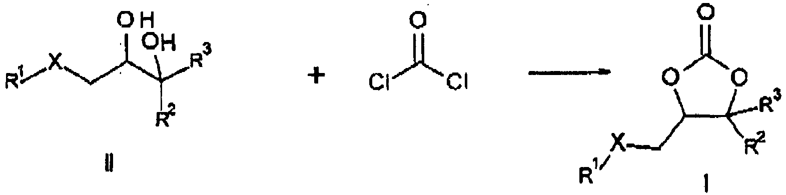

Die Bedeutung der Symbole X, R¹, R² und R³ entspricht der vorstehen genannten Bedeutung.

Die Umsetzung erfolgt in einer bevorzugten Ausführungsform durch Zusammengeben einer gekühlten Lösung von Phosgen in einem aromatischen Lösungsmittel, bevorzugt Toluol, zu einer gekühlten Lösung des mit einer R¹-X-CH₂-Gruppe funktionalisierten 1,2-Diols der Formel II in einem aromatischen Lösungsmittel, bevorzugt ebenfalls Toluol, in Anwesenheit einer Base, bevorzugt eines Amins, besonders bevorzugt Triethylamin oder Dimethylcyclohexylamin, zur Neutralisation von bei der Umsetzung gebildeter HCl. Die Temperatur sollte während des Zusammengebens 0°C nicht übersteigen. Sie beträgt bevorzugt -5°C bis 0°C. Nach Erwärmung der Reaktionsmischung auf Raumtemperatur wird die Umsetzung für im allgemeinen 1 bis 20 Stunden, bevorzugt 12 bis 16 Stunden bei Raumtemperatur weitergeführt. Nach Beendigung der Umsetzung erfolgt eine Aufarbeitung und anschließende Reinigung des gewünschten Alkylglycidolcarbonats nach dem Fachmann bekannten Methoden. Die bevorzugt eingesetzte Aminbase kann ggf. als Hydrochlorid isoliert und dem Verfahren nach Freisetzen des Amins und ggf. Abtrennung von Wasser wieder zugeführt werden.

Phosgen wird im allgemeinen in 0-50%igem molarem Überschuss, bevorzugt in 0-20%igem molaren Überschuss im Verhältnis zu dem Diol der Formel II eingesetzt. Dabei bedeutet ein 0 %iger Überschuss, dass Phosgen und das Diol in äquimolaren Mengen eingesetzt werden. Die eingesetzte Base wird im allgemeinen in einem molaren Verhältnis zu Phosgen von im allgemeinen 2 zu 1 bis 4 zu 1, bevorzugt 2 zu 1 bis 2,5 zu 1 eingesetzt.

Die 1,2-Diole der Formel II sind z.B. durch Epoxidierung eines geeigneten internen oder α-Olefins erhältlich, wobei ein Epoxid der Formel III erhalten wird:

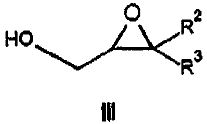

Einige der Epoxide der Formel III können auch käuflich erworben werden, z.B. Glycidol selbst.

Das Epoxid der Formel III wird anschliessend zu einem funktionalisierten Diol der Formel II umgesetzt:

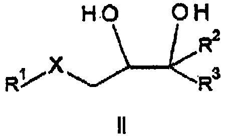

Die Bedeutung der Symbole X, R¹, R² und R³ entspricht der vorstehend genannten Bedeutung.

Die Funktionalisierung erfolgt z.B. durch Umsetzung des Epoxids III mit geeigneten Alkoholen, Thiolen, mit Alkylenoxiden umgesetzten Alkoholen, Aminen, Carbonsäuren oder deren Carbonsäureamiden. Geeignete Verbindungen dieser Gruppen werden nachstehend genannt.

Prinzipiell sind Diole der Formel II auch durch Hydrolyse von Epoxiden der allgemeinen Formel IV erhältlich.

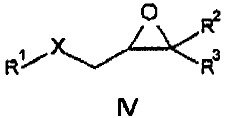

Die Hydrolyse des Epoxids der Formel IV führt zu den gewünschten Diolen der Formel II. Geeignete Hydrolysebedingungen sind dem Fachmann bekannt. Einige der geeigneten Diole können käuflich erworben werden.

Die Herstellung der Epoxide IV kann z.B. durch Umsetzung eines Nucleophils R1-XH mit Epichlorhydrin und anschließender HCl-Eliminierung erfolgen. Zur Umsetzung mit Epichlorhydrin wird ggf. ein saurer Katalysator zugesetzt. Die Eliminierung von HCl kann z. B. durch Vermischen des Umsetzungsproduktes aus Epichlorhydrin und Nucleophil mit wäßriger Natronlauge und ggf. Erwärmen erfolgen. Solche Umsetzungen sind dem Fachmann bekannt und in der gleichzeitig eingereichten Anmeldung mit dem Titel "Umsetzungsprodukte von 2-Propylheptanol" (DE-A 102 46 140) am Beispiel des 2-Propylheptanols als Nucleophil ausführlich beschrieben.

### b) CO₂-Insertion

Gemäß dieser Umsetzung werden die funktionalisierten Epoxide der Formel IV gemäß dem folgenden Reaktionsschema mit CO₂ unter Einsatz eines Katalysators umgesetzt (Paddock, Nguyen, J. Am. Chem. Soc. 2001, 123, 11498; Kisch, Millini, Wang, Chem. Ber. 1986, 119 (3), 1090; Baba, Nozaki, Matsuda, Bull. Chem. Soc. Jpn. 1987, 60 (4), 1552; Lermontov, Velikokhat'ko, Zavorin, Russ. Chem. Bull. 1998, 47 (7), 1405; Rokicki, Kuran, Pogorzelska-Marciniak, Monatshefte für Chemie 1984, 115, 205):

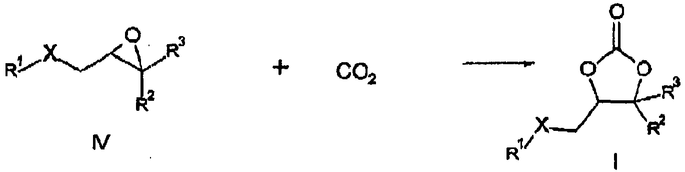

Die Bedeutung der Symbole X, R¹, R² und R³ entspricht der vorstehend genannten Bedeutung.

Die Herstellung der funktionalisierten Epoxide (IV) erfolgt wie vorstehend unter a) erwähnt. Bei der anschließenden Umsetzung mit CO₂ wird das Epoxid unter erhöhtem Druck von im allgemeinen 1 bis 50 bar, bevorzugt 1 bis 15 bar und erhöhter Temperatur von im allgemeinen 25 bis 150 °C, bevorzugt 40 bis 120 °C mit Kohlendioxid umgesetzt. Verfügbare Katalysatoren für die Umsetzung sind z. B. Amine, Übergangsmetall-Salen-Komplexe, Zink-Salze oder Kombinationen von Zink-Salzen mit quartären Ammoniumsalzen. Die anschließende Aufarbeitung und Reinigung des gewünschten Alkylglycidolcarbonats erfolgt nach dem Fachmann bekannten Methoden.

Zur Funktionalisierung der Epoxide der Formel III oder zur Umsetzung mit Epichlorhydrin geeignete Alkohole und Alkoholalkoxylate (mit Alkylenoxiden umgesetzte Alkohole) sind Verbindungen, durch die der Rest R¹ an das Epoxid addiert wird, so dass ein Epoxid der Formel IV oder - nach Hydrolyse - ein Diol der Formel II erhalten wird, die zu dem gewünschten Alkylglycidolcarbonat umgesetzt werden können. Geeignete Alkohole sind im folgenden aufgeführt.

### Alkohole:

Bei den geeigneten Alkoholen handelt es sich um lineare oder verzweigte aliphatische C₃-C₂₉-Akohole, vorzugsweise C₅-C₁₈-Alkohole. Diese Alkohole weisen gemäß Ausführungsform I einen mittleren Verzweigungsgrad von 0,2, bis 1,6 auf und gemäß Ausführungsform II einen mittleren Verzweigungsgrad von 0 bis 2,5, vorzugsweise 0,2 bis 1,6, auf. Der Verzweigungsgrad ist dabei definiert als. (Anzahl Methylgruppen pro Molekül) -1. Da der mit der Hydroxyfunktion verbundene aliphatische Kettenrest des Alkohols dem Rest R¹ in der Formel I entspricht, weist auch dieser letztgenannte Rest einen entsprechenden Verzweigungsgrad auf. An der Alkylkette sind keine weiteren Substituenten vorhanden. Beispiele für einsetzbare Alkohole umfassen Butanol, Pentanol, Hexanol, Heptanol, Octanol, Nonanol, Decanol, Undecanol, Dodecanol, Tridecanol, Tetradecanol und Hexadecanol. Von allen vorstehend genannten Alkoholen können sowohl die unverzweigte n-Form als auch verzweigte Isomere eingesetzt werden. Generell werden Isomerengemische der eingesetzten Alkohole verwendet, die den gewünschten mittleren Verzweigungsgrad aufweisen.

Bevorzugt eingesetzte Alkohole sind C₁₃H₂₇OH, C₁₅H₃₁OH, C₁₀H₂₁OH, C₁₆H₃₃OH, C₁₈H₃₇OH, C₁₂H₂₅OH, C₁₄H₂₉OH, C₈H₁₇OH.

Es ist auch möglich, Gemische von Alkoholen, unterschiedlicher C-Zahl einzusetzen und die daraus hergestellten Alkylglycidolcarbonatgemische als Co-Tenside zu verwenden. Diese Ausführungsform ist erfindungsgemäss bevorzugt. Besonders bevorzugt ist dabei der Einsatz technischer Mischungen von Alkoholen, insbesondere von Gemischen aus C9-/C11-Alkoholen, C₁₂-/C₁₄-Alkoholen, C₁₂-/C₁₅-Alkoholen, C₁₃-/C₁₅-Alkoholen und/oder C₁₆/C₁₈ Alkoholen.

Weiterhin ist der Einsatz sogenannter Guerbet-Alkohole und ihrer ungesättigten -Analoga erfingdungsgemäß bevorzugt. Es handelt sich hierbei um Alkohole mit einer Verzweigung in 2-Position. Beispiele umfassen 2-Ethylhexanol, 2-Ethylhex-2-enol, 2-Propylhexanol, 2-Propylheptanol, 2-Propylhept-2-enol, 2-Butyloctanol, 2-Butyloct-2-enol, 2-Pentylnonanol und 2-Pentylnon-2-enol. Gesättigte Alkohole sind bevorzugt.

Weiterhin sind auch sekundäre Alkohole oder Gemische, die diese Akohole enthalten, geeignet. Diese sind beispielsweise erhältlich nach einem der folgenden Verfahren:
1. Addition von Ketonen an Aldehyde mit nachfolgender Hydrierung wie in der DE 100 35 617.6 beschrieben, Bevorzugt sind Methylketone wie Aceton, Methylethylketon oder Methylisobutylketon..
2. Geeignet sind auch Paraffin-Oxidationsprodukte, die z.B. durch Bashkirov-Oxidation entstehen. Hier sind Produkte aus C11-C15 Paraffinen, besonders Produkte aus C12-C14-Paraffinen bevorzugt.
3. Addition von Wasser an Olefine
4. Radikalische oder sonstige Oxidation von Olefinen.

Die hier beschriebenen Alkohole liegen ebenso wie die im folgenden beschriebenen Nucleophile häufig nicht in Reinform, sondern als technische Gemische vor. So ist z.B, bein Einsatz von 2-Propylheptanol (C₁₀H₂₁OH) die Verwendung eines technischen Gemisches bevorzugt. In diesem technischen Gemisch liegt 2-Propylheptanol der allgemeinen Formel C₅H₁₁CH(C₃H₇)CH₂O als Gemisch mindestens zweier Isomeren vor, wobei 70 bis 99 Gew% Verbindungen enthalten sind, bei denen C₅H₁₁ die Bedeutung n-C₅H₁₁ hat und 1 bis 30 Gew% Verbindungen enthalten sind, bei denen C₅H₁₁ die Bedeutung C₂H₅CH(CH₃)CH₂ und/oder CH₃CH(CH₃)CH₂CH₂ hat.

### Alkoholalkoxylate:

Alkoholalkoxylate sind das Produkt der Polymerisationsreaktion von Alkoholen mit Alkylenoxiden z. B. Ethylenoxid, Propylenoxid, Butylenoxid, Pentylenoxid oder Gemischen davon.
Die Alkyl- und Alkenylrest der mit Alkylenoxiden umgesetzten Alkohole entsprechen ebenfalls den Alkyl- und Alkenylresten der vorstehend genannten geeigneten Alkohole und Alkobolmischungen.

Ganz besonders bevorzugt werden Alkohole eingesetzt.

Besonders bevorzugte Alkylglycidolcarbonate der allgemeinen Formel I sind Verbindungen gemäß Ausführungsform I, worin R² und R³ Wasserstoff bedeuten, X O ist und R1 ausgewählt ist aus C₁₃H₂₇, C₁₅H₃₁, C₁₀H₂₁, C₁₆H₃₃, C₁₈H₃₇, C₁₂H₂₅, C₁₄H₂₉, C₈H₁₇, C9-C11-Alkylresten, C₁₂-/C₁₄-Alkylresten, C12-/C15-Alkylresten, C13-/C15-Alkylresten und C16-/C18-Alkylresten.

Ganz besonders bevorzugt sind Alkylglycidolcarbonate der Struktur I gemäß Ausführungsform I, bei denen R¹ = C₅H₁₁CH(C₃H₇)CH₂O ist und R² sowie R³ = H sind und X=O. Dabei ist noch mehr bevorzugt, wenn es sich um ein Gemisch von Verbindungen handelt, in dem 70 bis 99 Gew% Verbindungen enthalten sind, bei denen C₅H₁₁ die Bedeutung n-C₅H₁₁ hat und 1 bis 30 Gew% Verbindungen enthalten sind, bei denen C₅H₁₁ die Bedeutung C₂H₅CH(CH₃)CH₂ und/oder CH₃CH(CH₃)CH₂CH₂ hat. Ebenfalls ganz besonders bevorzugt sind Alkylglycidolcarbonate der Struktur I gemäß Ausführungsform I, bei denen R1 ein technischer C13-/C15-Alkohol oder ein nativer oder technischer C12-C14-Alkohol oder ein technischer C10- oder C13-Alkohol mit Verzweigungsgrad ca. 1,5 ist und R2 und R3 = H sind und X=O.

Gemische zweier oder mehr der erfindungsgemäßen Alkylglycidolcarbonate sind ebenfalls Gegenstand der meistbevorzugten Ausführungsform der vorliegenden Erfindung.

Ein weiterer Gegenstand der Erfindung ist die Verwendung der Verbindungen der allgemeinen Formel I als Co-Tensid.

Die erfindungsgemäßen, als Co-Tenside zu verwendenden Substanzen der Formel I eigenen sich zum Einsatz in industriellen, institutionellen oder Haushaltswaschmitteln und -reinigern sowie im sogenannten Body-Care-Sektor, also Körperreinigungs- und -pflegemitteln.

Weitere Anwendungen sind:
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Formulierungen für die Textilindustrie wie Egalisiermittel oder Formulierungen zur Garnreinigung.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Die Waschmittel liegen in fester, flüssiger, gelförmiger oder pastenförmiger Form vor. Zu den in fester Form vorliegenden Materialien gehören Pulver und Kompaktate, beispielsweise Granulate und Formkörper wie Tabletten.

Die Waschmittel enthalten 0,1 bis 40 Gew-%, insbesondere 0,5 bis 30 Gew-%, ganz besonders 1 bis 20 Gew-%, bezogen auf die Gesamtmenge der Formulierung, mindestens einer Substanz der Formeln I und/oder II. Weitere Bestandteile sind nachfolgend aufgeführt.

Waschmittelformulierungen enthalten üblicherweise Inhaltsstoffe wie Tenside, Gerüst-, Duft- und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 beispielhaft beschrieben.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen.

Es kann sich um Rohfasern oder um Fasern in beliebigen Verarbeitungsstadien handeln und es können natürliche Eiweiß- oder Zellulosefasern wie Wolle, Seide, Baumwolle, Sisal, Hanf, Kokosfasern oder Synthesefasern wie beispielsweise Polyester-, Polyamid- oder Polyacrylnitrilfasern sein.

Waschmittel enthaltend die erfindungsgemässen Co-Tenside können auch zur Reinigung von faserhaltigen Materialien, wie z. B. rückenbeschichteten Teppichen mit geschnittenem oder ungeschnittenem Flor dienen.

Die Zusammensetzungen der Waschmittel werden vorzugsweise den verschiedenen Zwecken angepasst, wie es dem Fachmann aus dem Stand der Technik geläufig ist. Hierzu können den Waschmitteln alle zweckentsprechenden aus dem Stand der Technik bekannten Hilfs- und Zusatzstoffe zugefügt werden.

In Waschmitteln können neben den erfindungsgemäßen Co-Tensiden beispielsweise vorliegen:
- Builder und Cobuilder, wie Polyphosphate, Zeolithe, Polycarboxylate, Phosphonate oder Komplexbildner
- ionische Tenside, wie Alkoholsulfate/-ethersulfate, Alkylbenzolsulfonate, α-Olefinsulfonate und andere Alkoholsulfate/-ethersulfate
- nichtionische Tenside, Alkoholalkoxylate wie Alkylaminalkoxylate, Alkylpolyglucoside
- optische Aufheller
- Farbübertragungsinhibitoren, wie Polyvinylpyrrolidon der Molmassen 8.000 bis 70.000, Vinylimidazol/Vinylpyrrolidon-Copolymere mit einem Molverhältnis der Monomeren von 1:10 bis 2:1 und Molmassen von 8.000 bis 70.000 sowie Poly-4-vinylpyridin-N-oxide mit Molmassen von 8.000 bis 70.000.
- Stellmittel, wie Natriumsulfat oder Magnesiumsulfat
- Soil Release-Mittel
- Inkrustationsinhibitoren
- Bleichsysteme, enthaltend Bleichmittel, wie Perborat, Percarbonat und Bleichaktivatoren, wie Tetraacetylethylendiamin sowie Bleichstabilisatoren
- Parfum/-öle
- Schaumdämpfer, wie Silikonöle
- Enzyme, wie Amylasen, Lipasen, Cellulasen, Proteasen
- Alkalispender, wie lösliche Alkalisilikate, z. B. Pentanatriummethasilikat, Nariumcarbonat.

In flüssigen Waschmitteln können beispielsweise zusätzlich Lösungsmittel, wie Ethanol, Isopropanol, 1,2-Propylenglycol, Butylglycol usw. eingesetzt werden.

In tablettenförmigen Waschmitteln können zusätzlich Tablettierhilfsmittel, wie Polyethylenglycole mit Molmassen von mehr als 1000 g/mol, Polymerdispersionen sowie Tablettensprengmittel, wie Cellulosederivate, vernetztes Polyvinylpyrrolidon, vernetzte Polyacrylate oder Kombinationen aus Säuren, wie Zitronensäure und Natriumbicarbonat eingesetzt werden. Eine detailliertere Aufführung möglicher Inhaltsstoffe wird nachfolgend gegeben.

In manchen Fällen kann es zweckmäßig sein, die erfindungsgemäß eingesetzten Co-Tenside mit anderen Co-Tensiden oder mit amphoteren Tensiden, wie z. B. Alkylaminoxiden, oder Betainen zu kombinieren.

Eine andere Klasse nichtionischer Tenside sind Alkylpolyglucoside mit 6 bis 22, vorzugsweise 10 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Strukturen 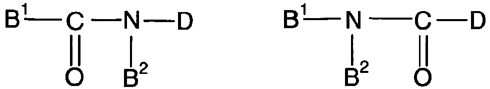 wobei B¹ ein C₆- bis C₂₂-Alkyl, B² Wasserstoff oder C₁- bis C₄-Alkyl und D ein Polyhydroxyalkyl-Rest mit 5 bis 12 C-Atomen und mindestens 3 Hydroxygruppen ist. Vorzugsweise steht B¹ für C₁₀- bis C₁₈-Alkyl, B² für CH₃ und D für einen C₅- oder C₆-Rest. Beispielsweise erhält man derartige Verbindungen durch die Acylierung von reduzierend aminierten Zuckern mit Säurechloriden von C₁₀- bis C₁₈-Carbonsäuren.

Weitere in Betracht kommende nichtionische Tenside sind die aus der WO-A 95/11225 bekannten endgruppenverschlossenen Fettsäureamidalkoxylate der allgemeinen Formel

R¹-CO-NH- (CH₂)_{y}-O- (A¹O)ₓ-R²

in der
- R¹: einen C₅- bis C₂₁-Alkyl- oder Alkenylrest bezeichnet,
- R²: eine C₁- bis C₄-Alkylgruppe bedeutet,
- A¹: für C₂- bis C₄-Alkylen steht,
- y: die Zahl 2 oder 3 bezeichnet und
- x: einen Wert von 1 bis 6 hat.

Beispiele für solche Verbindungen sind die Umsetzungsprodukte von n-Butyltriglykolamin der Formel H₂N-(CH₂-CH₂-O)₃-C₄H₉ mit Dodecansäuremethylester oder die Reaktionsprodukte von Ethyltetraglykolamin der Formel H₂N-(CH₂-CH₂-O)₄-C₂H₅ mit einem handelsüblichen Gemisch von gesättigten C₈- bis C₁₈-Fettsäuremethylestern.

Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die zusätzlichen nichtionischen Tenside ("Niotenside") liegen in den Waschmitteln enthaltend die erfindungsgemäss verwendetenen Co-Tenside vorzugsweise in einer Menge von 0,01 bis 30 Gew.-%, insbesondere 0,1 bis 25 Gew.-%, vor allem 0,5 bis 20 Gew.-%, vor.

Man kann zusätzlich einzelne nichtionische Tenside oder eine Kombination unterschiedlicher Niotenside einsetzen. Es können nichtionische Tenside aus nur einer Klasse zum Einsatz gelangen, insbesondere nur alkoxylierte C₈- bis C₂₂-Alkohole, man kann aber auch Tensidmischungen aus verschiedenen Klassen verwenden.

Geeignete anionische Tenside sind beispielsweise Fettalkoholsulfate von Fettalkoholen mit 8 bis 22, vorzugsweise 10 bis 18 Kohlenstoffatomen, C₁₂-C₁₈-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, dass man zunächst einen C₈- bis C₂₂-, vorzugsweise einen C₁₀- bis C₁₈-Alkohol z. B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid oder Ethylenoxid und Propylenoxid und Butylenoxid enthalten. Die alkoxylierten C₈- bis C₂₂-Alkohole können die Ethylenoxid-, Propylenoxid- und Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie C₈- bis C₂₄-, vorzugsweise C₁₀- bis C₁₈-Alkansulfonate sowie Seifen wie beispielsweise die Na- und K-Salze von gesättigten und/oder ungesättigten C₈- bis C₂₄-Carbonsäuren.

Weitere geeignete anionische Tenside sind lineare C₈- bis C₂₀-Alkylbenzolsulfonate ("LAS"), vorzugsweise lineare C₉- bis C₁₃-Alkylbenzolsulfonate und -Alkyltoluolsulfonate.

Weiterhin eignen sich als anionische Tenside noch C₈- bis C₂₄-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobernsteinsäuren oder deren Amide, Mono- und Diester von Sulfobemsteinsäuren, Acylsarkosinate, sulfatierte Alkylpolyglucoside, Alkylpolyglykolcarboxylate sowie Hydroxyalkylsarkosinate.

Die anionischen Tenside werden dem Waschmittel vorzugsweise in Form von Salzen zugegeben. Geeignete Kationen in diesen Salzen sind Alkalimetallionen wie Natrium, Kalium und Lithium und Ammoniumsalze wie z. B. Hydroxyethylammonium-, Di(hydroxyethyl)ammonium- und Tri(hydroxyethyl)ammoniumsalze.

Die anionischen Tenside liegen in den Waschmitteln enthaltend die erfindungsgemässen Co-Tenside vorzugsweise in einer Menge von bis zu 30 Gew.-%, beispielsweise von 0,1 bis 30 Gew.-%, vor allem 1 bis 25 Gew.%, insbesondere 3 bis 10 Gew.-% vor. Werden C₉- bis C₂₀-linear-Alkyl-benzolsulfonate (LAS) mitverwendet, kommen diese üblicherweise in einer Menge bis zu 15 Gew.-%, insbesondere bis zu 10 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensidmischungen aus verschiedenen Klassen verwenden, z. B. eine Mischung aus Fettalkoholsulfaten und Alkylbenzolsulfonaten.

Ferner können Tensidgemische enthaltend die erfindungsgemäss einzusetzenden Co-Tenside mit kationischen Tensiden, üblicherweise in einer Menge bis 25 Gew.-%, vorzugsweise 1 bis 15 Gew.-%, beispielsweise C₈-bis C₁₆-Dialkyldimethylammoniumsalzen, Dialkoxydimethyl-ammoniumsalzen oder Imidazoliniumsalzen mit langkettigem Alkylrest; und/oder mit amphoteren Tensiden, üblicherweise in einer Menge bis 15 Gew.-%, vorzugsweise 1 bis 10 Gew.-%, beispielsweise Derivaten von sekundären oder tertiären Aminen wie z. B. C₆-C₁₈-Alkylbetainen oder C₆-C₁₅-Alkylsulfobetainen oder Alkylamidobetainen oder Aminoxiden wie Alkyldimethylaminoxiden kombiniert werden. Ferner können kationische Tenside eingesetzt werden, wie sie in der WO 99/19435 beschrieben sind.

In der Regel werden Gemische enthaltend die erfindungsgemäss einzusetzenden Co-Tenside mit Buildern (Sequestrierungsmitteln) wie z. B. Polyphosphaten, Polycarboxilaten, Phosphonaten, Komplexbildnern, z. B. Methylglycindiessigsäure und deren Salze, Nitrilotriessigsäure und deren Salze, Ethylendiamintetraessigsäure und deren Salze sowie gegebenenfalls mit Co-Buildern kombiniert.

Einzelne zur Kombination mit Gemischen enthaltend die erfindungsgemäss einzusetzenden Co-Tenside gut geeignete Buildersubstanzen seien im Folgenden aufgezählt:
Geeignete anorganische Builder sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4,604,224.

Als Builder geeignete kristalline Silicate sind beispielsweise Disilicate oder Schichtsilicate, z. B. δ-Na₂Si₂O₅ oder ß-Na₂Si₂O₅. Die Silicate können in Form ihrer Alkalimetall-, Erdalkalimetall- oder Ammoniumsalze eingesetzt werden, vorzugsweise als Na-, Li- und Mg-Silicate.Amorphe Silicate wie beispielsweise Natriummetasilicat, welches eine polymere Struktur aufweist, oder amorphes Disilicat sind ebenfalls verwendbar.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. - Hydrogencarbonate, insbesondere Natriumcarbonat und/oder Natriumhydrogencarbonat, eingesetzt.

Übliche, als anorganische Builder eingesetzte Phosphate sind Alkaliorthophosphate,und/oder -Polyphosphate wie z. B. Pentanatriumtriphosphat. Die genannten Builder-Komponenten können einzeln oder in Mischungen untereinander eingesetzt werden.

Ferner ist es in vielen Fällen zweckmäßig den Waschmitteln enthaltend die erfindungsgemäss einzusetzenden Co-Tenside Co-Builder zuzufügen. Beispiele für geeignete Substanzen sind im Folgenden aufgelistet:
In einer bevorzugten Ausführungsform enthalten die Waschmittel enthaltend die erfindungsgemäss einzusetzenden Co-Tenside zusätzlich zu den anorganischen Buildern 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:
Phosphonsäuren wie z. B. 1-Hydroxyethan-1,1-diphosphonsäure, Amino-tris(methylenphosphonsäure), Ethylendiamin-tetra(methylenphosphonsäure), Hexamethylendiamintetra(methylenphosphonsäure) und Diethylentriamin-penta(methylenphosphonsäure);
C₄-bis C₂₀-Di-, -Tri- und -Tetracarbonsäuren wie z. B. Bernsteinsäure, Propantricarbonsäure, Butantetracarbonsäure, Cyclopentantetracarbonsäure und Alkyl- und Alkenylbernsteinsäuren mit C₂- bis C₁₆-Alkyl- bzw. -Alkenyl-Resten;
C₄- bis C₂₀-Hydroxycarbonsäuren wie z. B. Äpfelsäure, Weinsäure, Gluconsäure, Glutarsäure, Citronensäure, Lactobionsäure und Saccharosemono-, di- und tricarbonsäure;
Aminopolycarbonsäuren wie z. B. Nitrilotriessigsäure, ß-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:
Oligomaleinsäuren, wie sie beispielsweise in EP-A 451 508 und EP-A 396 303 beschrieben sind;
Co- und Terpolymere ungesättigter C₄- bis C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können.

Als ungesättigte C₄- bis C₈-Dicarbonsäuren sind hierbei beispielsweise Maleinsäure, Fumarsäure, Itaconsäure und Citraconsäure geeignet. Bevorzugt wird Maleinsäure.

Die Gruppe (i) umfasst monoethylenisch ungesättigte C₃-C₈-Monocarbonsäuren wie z. B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte C₂- bis C₂₂-Olefine, Vinylalkylether mit C₁- bis C₈-Alkylgruppen, Styrol, Vinylester von C₁- bis C₈-Carbonsäuren, (Meth)acrylamid und Vinylpyrrolidon. Bevorzugt werden aus der Gruppe (ii) C₂- bis C₆-Olefine, Vinylalkylether mit C₁- bis C₄-Alkylgruppen, Vinylacetat und Vinylpropionat eingesetzt.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3,887,806 sowie DE-A 43 13 909 bekannt.

Die Gruppe (iii) umfasst (Meth)acrylester von C₁- bis C₈-Alkoholen, (Meth)acrylnitril, (Meth)acrylamide von C₁- bis C₈-Aminen, N-Vinylformamid und N-Vinylimidazol.

Als organische Cobuilder eignen sich auch Homopolymere der monoethylenisch ungesättigten C₃-C₈-Monocarbonsäuren wie z. B. Acrylsäure, Methacrylsäure, Crotonsäure und Vinylessigsäure, insbesondere der Acrylsäure und Methacrylsäure,
Copolymere von Dicarbonsäuren, wie z. B. Copolymere von Maleinsäure und Acrylsäure im Gewichtsverhältnis 10:90 bis 95:5, besonders bevorzugt solche im Gewichtsverhältnis 30:70 bis 90: 10 mit Molmassen von 1000 bis 150000;
Terpolymere aus Maleinsäure, Acrylsäure und einem Vinylester einer C₁-C₃-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5,227,446, DE-A 44 15 623 und DE-A 43 13 909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z. B. sauer oder enzymatisch abgebaute Stärken, Inuline oder Zellulose, Eiweißhydrolysate und reduzierte (hydrierte oder hydrierend aminierte) abgebaute Polysaccharide wie z. B. Mannit, Sorbit, Aminosorbit und N-Alkylglucamin geeignet sowie auch Polyalkylenglycole mit Molmassen mit bis zu M_{w} = 5000 wie z. B. Polyethylenglycole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige C₁- bis C₂₂-Alkohole.(vgl. US-A-5,756,456)

Als organische Cobuilder geeignete Polyglyoxylsäuren sind beispielsweise beschrieben in EP-B-001 004, US-A-5,399,286, DE-A-41 06 355 und EP-A-656 914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A-454 126, EP-B-511 037, WO-A-94/01486 und EP-A-581452.

Als organische Cobuilder verwendet man insbesondere auch Polyasparaginsäuren oder Cokondensate der Asparaginsäure mit weiteren Aminosäuren, C₄- bis C₂₅-Mono- oder - Dicarbonsäuren und/oder C₄- bis C₂₅-Mono- oder -Diaminen. Besonders bevorzugt werden in phosphorhaltigen Säuren hergestellte, mit C₆- bis C₂₂-Mono- oder -Dicarbonsäuren bzw. mit C₆- bis C₂₂-Mono- oder -Diaminen modifizierte Polyasparaginsäuren eingesetzt.

Als organische Cobuilder eignen sich weiterhin Iminodibernsteinsäure, Oxydibemsteinsäure, Aminopolycarboxylate, Alkylpolyaminocarboxylate, Aminopolyalkylenphosphonate, Polyglutamate, hydrophob modifizierte Citronensäure wie z. B. Agaricinsäure, Poly-α-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Cobuilder verwendet werden.

Weitere geeignete (Co)builder sind in WO 99/19435 beschrieben.

In einer weiteren bevorzugten Ausführungsform enthalten die Waschmittel enthaltend die erfindungsgemäss einzusetzenden Co-Tenside zusätzlich, insbesondere zusätzlich zu den anorganischen Buildem, den anionischen Tensiden und/oder den nichtionischen Tensiden, 0,5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den Waschmitteln enthaltend die erfindungsgemäss einzusetzenden Co-Tenside Bleichsysteme, bestehend aus Bleichmitteln, wie z. B. Perborat, Percarbonat und gegebenenfalls Bleichaktivatoren, wie z. B. Tetraacetylethylendiamin, + Bleichstabilisatoren sowie gegebenenfalls Bleichkatalysatoren zuzusetzen.

In diesen Fällen enthalten die Waschmittel enthaltend die erfindungsgemäss einzusetzenden Co-Tenside zusätzlich 0,5 bis 30 Gew.-%, insbesondere 5 bis 27 Gew.-%, vor allem 10 bis 23 Gew.-% Bleichmittel in Form von Percarbonsäuren, z. B. Diperoxododecandicarbonsäure, Phthalimidopercapronsäure oder Monoperoxophthalsäure oder - terephthalsäure, Addukten von Wasserstoffperoxid an anorganische Salze, z. B. Natriumperborat-Monohydrat, Natriumperborat-Tetrahydrat, Natriumcarbonat-Perhydrat oder Natriumphosphat-Perhydrat, Addukten von Wasserstoffperoxid an organische Verbindungen, z. B. Harnstoff-Perhydrat, oder von anorganischen Peroxosalzen, z. B. Alkalimetallpersulfaten, oder -peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren.

Als Bleichaktivatoren eignen sich:
- polyacylierte Zucker, z. B. Pentaacetylglucose;
- Acyloxybenzolsulfonsäuren und deren Alkali- und Erdalkaiimetallsalze, z. B. Natrium-p-nonanoyloxybenzolsulfonat oder Natrium-p-benzoyloxybenzolsulfonat;
- N,N-diacylierte und N,N,N',N'-tetraacylierte Amine, z. B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z. B. N-Methyl-N-mesylacetamid oder N-Methyl-N-mesylbenzamid;
- N-acylierte cyclische Hydrazide, acylierte Triazole oder Urazole, z. B. Monoacetylmaleinsäurehydrazid;
- O,N,N-trisubstituierte Hydroxylamine, z. B. O-Benzoyl-N,N-succinylhydroxylamin, O-Acetyl-N,N-succinylhydroxylamin oder O,N,N-Triacetylhydroxylamin;
- N,N'-Diacylsulfurylamide, z. B. N,N'-Dimethyl-N,N'-diacetylsulfurylamid oder N,N'-Diethyl-N,N'-dipropionyisulfurylamid;
- acylierte Lactame wie beispielsweise Acetylcaprolactam, Octanoylcaprolactam, Benzoylcaprolactam oder Carbonylbiscaprolactam;
- Anthranilderivate wie z. B. 2-Methylanthranil oder 2-Phenylanthranil;
- Triacylcyanurate, z. B. Triacetylcyanurat oder Tribenzoylcyanurat;
- Oximester und Bisoximester wie z. B. O-Acetylacetonoxim oder Bisisopropyliminocarbonat;
- Carbonsäureanhydride, z. B. Essigsäureanhydrid, Benzoesäureanhydrid, m-Chlorbenzoesäureanhydrid oder Phthalsäureanhydrid;
- Enolester wie z. B. Isopropenylacetat;
- 1,3-Diacyl-4,5-diacyloxy-imidazoline, z. B. 1,3-Diacetyl-4,5-diacetoxyimidazolin;
- Tetraacetylglycoluril und Tetrapropionylglycoluril; diacylierte 2,5-Diketopiperazine, z. B. 1,4-Diacetyl-2,5-diketopiperazin;
- ammoniumsubstituierte Nitrile wie z. B. N-Methylmorpholiniumacetonitrilmethylsulfat;
- Acylierungsprodukte von Propylendiharnstoff und 2,2-Dimethyl-propylendiharnstoff, z. B. Tetraacetylpropylendiharnstoff;
- α-Acyloxypolyacylmalonamide, z. B. α-Acetoxy-N,N'-diacetylmalonamid;
- Diacyl-dioxohexahydro-1,3,5-triazine, z. B. 1,5-Diacetyl-2,4-dioxohexahydro-1,3,5-triazin;
- Benz-(4H)1,3-oxazin-4-one mit Alkylresten, z. B. Methyl, oder aromatischen Resten z. B. Phenyl, in der 2-Position;
- kationische Nitrile, wie in DE-A-101 48 577 beschrieben.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5,360,569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Waschmitteln höchstens in Mengen bis 1,5 Gew.-%, insbesondere bis 0,5 % Gew.-%, im Falle von sehr aktiven Mangankomplexen in Mengen bis zu 0,1 Gew.-%, eingearbeitet. Weitere geeignete Bleichkatalysatoren sind in WO 99/19435 beschrieben.

Weitere einsetzbare Bleichsysteme auf Basis von Arylimidoperalkansäuren sind in EP-A-0 325 288 und EP-A-0 490 409 beschrieben.

### Bleichstabilisator

Dabei handelt es sich um Additive, die Schwermetallspuren absorbieren, binden oder komplexieren können. Beispiele für erfindungsgemäß verwendbare Zusätze mit bleichstabilisierender Wirkung sind polyanionische Verbindungen wie Polyphosphate, Polycarboxylate, Polyhydroxypolycarboxylate, lösliche Silikate als vollständig oder teilweise neutralisierte Alkali- oder Erdalkalisalze, insbesondere als neutrale Na- oder Mg-Salze, die relativ schwache Bleichstabilisatoren sind. Starke erfindungsgemäß verwendbare Bleichstabilisatoren sind beispielsweise Komplexbildner, wie Ethylendiamintetraacetat (EDTA), Nitrilotriessigsäure (NTA), Methylglycindiessigsäure (MGDA), β-Alanindiessigsäure (ADA), Ethylendiamin-N,N'-diesuccinat (EDDS) und Phosphonate wie Ethylendiamintetramethylenphosphonat, Diethylentriaminpentamethylenphosphonat oder Hydroxyethyliden-1,1-diphosphonsäure in Form der Säuren oder als teilweise oder vollständig neutralisierte Alkalimetallsalze. Vorzugsweise werden die Komplexbildner in Form ihrer Na-Salze eingesetzt.

Neben dem beschriebenen Bleichsystem aus Bleichmitteln, Bleichaktivatoren und gegebenenfalls Bleichkatalysatoren ist für Waschmittel enthaltend die erfindungsgemäss einzusetzenden Co-Tenside auch die Verwendung von Systemen mit enzymatischer Peroxidfreisetzung oder von photoaktivierten Bleichsystemen möglich, siehe z. B. US 4,033,718.

Für eine Reihe von Anwendungsfällen ist es zweckmäßig, wenn die Waschmittel enthaltend die erfindungsgemäss einzusetzenden Co-Tenside Enzyme enthalten. Vorzugsweise in Waschmitteln eingesetzte Enzyme sind Proteasen, Amylasen, Lipasen und Cellulasen. Von den Enzymen werden vorzugsweise mengen von 0,1 bis 1,5 Gew.-%, insbesondere vorzugsweise 0,2 bis 1,0 Gew.-%, des konfektionierten Enzyms zugesetzt. Geeignete Proteasen sind z. B. Savinase und Esperase. Eine geeignete Lipase ist z. B. Lipolase. Eine geeignete Cellulase ist z. B. Celluzym. Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das Waschmittel enthaltend die erfindungsgemäss einzusetzenden Co-Tenside noch Enzymstabilisatoren, z. B. Calciumpropionat, Natriumformiat oder Borsäuren oder deren Salze, und/oder Oxidationsverhinderer enthalten.

Die Bestandteile von Waschmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die Waschmittel enthaltend die erfindungsgemäss einzusetzenden Co-Tenside können neben den bisher genannten Hauptkomponenten noch folgende weitere übliche Zusätze in den hierfür üblichen Mengen enthalten:
Bekannte Dispergiermittel, wie Naphthalinsulfonsäurekondensate oder Polycarboxilate, Schmutztragemittel, Soil release Agentien, wie Polyetherester, Inkrustationsinhibitoren, pH-regulierende Verbindungen wie Alkalien bzw. Alkalispender (NaOH, KOH, Pentanatriummetasilikat, Natriumcarbonat) oder Säuren (Salzsäure, Phosphorsäure, Amidoschwefelsäure, Citronensäure) Puffersysteme, wie Acetat oder Phosphatpuffer, Ionenaustauscher, Parfüm, Farbstoffe, Vergrauungsinhibitoren, optische (fluoreszierende) Aufheller, Farbübertragungsinhibitoren wie z. B. Polyvinylpyrrolidon, Biozide, wie Isothiazolinone oder 2-Bromo-2-nitro-1,3-propandiol, hydrotrope Verbindungen als Lösungsvermittler bzw. Solubilisatoren, wie Cumolsulfonate, Toluolsulfonate, kurzkettige Fettsäuren, Harnstoff, Alkohole oder Phosphorsäurealkyl/-arylester, Schaumregulatoren zur Stabilisierung oder Dämpfung des Schaums, z. B. Siliconöle, Haut- und Korrosionsschutzmittel, desinfizierende Verbindungen oder Systeme, wie z. B. solche die Chlor oder unterchlorige Säure freisetzen wie Dichlorisocyanurat oder die Iod enthalten, Verdickungsmittel und Stell- und Konfektionierungsmittel.

### Vergrauungsinhibitoren und Soil-Release-Polymere

Geeignete Soil-Release-Polymere und/oder Vergrauungsinhibitoren für Waschmittel sind beispielsweise:
Polyester aus Polyethylenoxiden mit Ethylenglycol und/oder Propylenglycol und aromatischen Dicarbonsäuren oder aromatischen und aliphatischen Dicarbonsäuren;
Polyester aus einseitig endgruppenverschlossenen Polyethylenoxiden mit zwei-und/oder mehrwertigen Alkoholen und Dicarbonsäure.

Derartige Polyester sind bekannt, beispielsweise aus US-A 3,557,039, GB-A 1 154 730, EP-A-185 427, EP-A-241 984, EP-A-241985, EP-A- 272 033 und US-A 5,142,020.

Weitere geeignete Soil-Release-Polymere sind amphiphile Pfropf- oder Copolymere von Vinyl- und/oder Acrylestern auf Polyalkylenoxide (vgl. US-A 4,746,456, US-A 4,846,995, DE-A-37 11 299, US-A 4,904,408, US-A 4,846,994 und US-A 4,849,126) oder modifizierte Cellulosen wie z. B. Methylcellulose, Hydroxypropylcellulose oder Carboxymethylcellulose.

### Farbübertragungsinhibitoren

Als Farbübertragungsinhibitoren werden beispielsweise Homo- und Copolymere des Vinylpyrrolidons, des Vinylimidazols, des Vinyloxazolidons und des 4-Vinylpyridin-N-oxids mit Molmassen von 15.000 bis 100.000 sowie vernetzte feinteilige Polymere auf Basis dieser Monomeren eingesetzt. Die hier genannte Verwendung solcher Polymere ist bekannt, vgl. DE-B- 22 32 353, DE-A-28 14 287, DE-A-28 14 329 und DE-A-43 16 023.

Geeignete Polyvinylpyridinbetaine sind z. B. in Tai, Formulating Detergents and Personal Care Products, AOCS Press, 2000, Seite 113 beschrieben.

Neben der Anwendung in Wasch- und Reinigungsmitteln für die Textilwäsche im Haushalt sind die erfindungsgemäß verwendbaren Waschmittelzusammensetzungen auch im Bereich der gewerblichen Textilwäsche und der gewerblichen Reinigung einsetzbar. In der Regel wird in diesem Einsatzbereich Peressigsäure als Bleichmittel eingesetzt, die als wäßrige Lösung der Waschflotte zugesetzt wird.

### Verwendung in Textilwaschmitteln

Ein typisches erfindungsgemäß einzusetzendes pulver- oder granulatförmiges Vollwaschmittel kann beispielsweise folgende Zusammensetzung aufweisen:
- 0,5 bis 50 Gew.-%, vorzugsweise 5 bis 30 Gew.-%, mindestens eines anionischen und/- oder nichtionischen Tensids, einschließlich mindestens eines erfindungsgemässen Co-Tensids,
- 0,5 bis 60 Gew.-%, vorzugsweise 15 bis 40 Gew.-%, mindestens eines anorganischen Builders,
- 0 bis 20 Gew.-%, vorzugsweise 0,5 bis 8 Gew.-%, mindestens eines organischen Cobuilders,
- 2 bis 35 Gew.-%, vorzugsweise 5 bis 30 Gew.-%, eines anorganischen Bleichmittels,
- 0,1 bis 20 Gew.-%, vorzugsweise 0,5 bis 10 Gew.-%, eines Bleichaktivators, gegebenenfalls in Abmischung mit weiteren Bleichaktivatoren,
- 0 bis 1 Gew.-%, vorzugsweise bis höchstens 0,5 Gew.-%, eines Bleichkatalysators,
- 0 bis 5 Gew.-%, vorzugsweise 0 bis 2,5%, eines polymeren Farbübertragungsinhibitors,
- 0 bis 1,5 Gew.-%, vorzugsweise 0,1 bis 1,0 Gew.-%, Protease,
- 0 bis 1,5 Gew.-%, vorzugsweise 0,1 bis 1,0 Gew.-%, Lipase,
- 0 bis 1,5 Gew.-%, vorzugsweise 0,2 bis 1,0% Gew.-% eines Soil-Release-Polymers,
ad 100% übliche Hilfs- und Begleitstoffe und Wasser.

Vorzugsweise in Waschmitteln eingesetzte anorganische Builder sind Natriumcarbonat, Natriumhydrogencarbonat, Zeolith A und P sowie amorphe und kristalline Na-Silikate sowie Schichtsilikate.

Vorzugsweise in Waschmitteln eingesetzte organische Cobuilder sind Acrylsäure/MaleinsäureCopolymere, Acrylsäure/Maleinsäure/Vinylester- Terpolymere und Citronensäure.

Vorzugsweise in Waschmitteln eingesetzte anorganische Bleichmittel sind Natriumperborat und Natriumcarbonat-Perhydrat.

Vorzugsweise in Waschmitteln eingesetzte anionische Tenside sind lineare und leicht verzweigte Alkylbenzolsulfonate (LAS), Fettalkoholsulfate/ethersulfate und Seifen.

Vorzugsweise in Waschmitteln eingesetzte Enzyme sind Protease, Lipase, Amylase und Cellulase. Von den handelsüblichen Enzymen werden dem Waschmittel in der Regel Mengen von 0,05 bis 2,0 Gew.-%, vorzugsweise 0,2 bis 1,5 Gew.-%, des konfektionierten Enzyms zugesetzt. Geeignete Proteasen sind z.B Savinase, Desazym und Esperase. Eine geeignete Lipasen ist z. B. Lipolase. Eine geeignete Cellulase ist z. B. Celluzym.

Vorzugsweise in Waschmitteln eingesetzte Vergrauungsinhibitoren und Soil-Release-Polymere sind Pfropfpolymere von Vinylacetat auf Polyethylenoxid der Molmasse 2.500-8.000 im Gewichtsverhältnis 1,2:1 bis 3,0:1, Polyethylen-terephthalate/Oxyethylenterephthalate der Molmasse 3.000 bis 25.000 aus Polyethylenoxiden der Molmasse 750 bis 5.000 mit Terephthalsäure und Ethylenoxid und einem Molverhältnis von Polyethylenterephthalat zu Polyoxyethylenterephthalat von 8:1 bis 1:1 sowie Blockpolykondensate gemäß DE-A-44 03 866.

Vorzugsweise in Waschmitteln eingesetzte Farbübertragungsinhibitoren sind lösliche NVP-Homopolymere und/oder Vinylpyrrolidon- und Vinylimidazol-Copolymere mit Molmassen über 5.000.

Die Waschmittel liegen häufig in fester, pulverförmiger Form vor, und enthalten dann in der Regel zusätzlich übliche Stellmittel, die ihnen eine gute Rieselfähigkeit, Dosierbarkeit und Löslichkeit verleihen und die das Zusammenbacken und Stauben verhüten, wie Natriumsulfat oder Magnesiumsulfat.

Pulver- oder granulatförmige Waschmittel enthaltend die erfindungsgemäss einzusetzenden Co-Tenside können bis zu 60 Gew.-% anorganischer Stellmittel enthalten. Vorzugsweise sind diese Waschmittel aber arm an Stellmitteln und enthalten nur bis zu 20 Gew.-%, besonders bevorzugt nur bis 8 Gew.-% an Stellmitteln.

Waschmittel enthaltend die erfindungsgemäss einzusetzenden Co-Tenside können unterschiedliche Schüttdichten im Bereich von 300 bis 1.200, insbesondere 500 bis 950g/l, besitzen. Moderne Kompaktwaschmittel besitzen in der Regel hohe Schüttdichten und zeigen einen Granulataufbau. Kompakt- oder Ultra-Kompaktwaschmittel sowie Extrudate weisen ein Schüttgewicht > 600 g/l auf. Diese gewinnen immer mehr an Bedeutung.

Sofern sie in flüssiger Form eingesetzt werden sollen, können sie als wässrige Mikroemulsionen, Emulsionen oder Lösungen vorliegen. In flüssigen Waschmitteln können zusätzlich Lösungsmittel wie Ethanol, i-Propanol, 1,2-Propylenglykol, oder Butylglykol verwendet werden.

Bei gelförmigen Waschmitteln können zusätzlich Verdicker, wie z. B. Polysaccharide und/oder schwach vernetzte Polycarboxylate (beispielsweise Carbopol® der Fa. Goodrich) eingesetzt werden.

Bei tablettenförmigen Waschmitteln werden zusätzlich Tablettierhilfsmittel wie z. B. Polyethylenglykole mit Molmassen > 1000 g/mol, Polymerdispersionen, und Tablettensprengmittel wie Cellulosederivate, vernetztes Polyvinylpyrrolidon, vernetzte Polyacrylate oder Kombinationen aus Säuren, z. B. Citronensäure + Natriumbicarbonat, um nur einige zu nennen, benötigt.

Ein weiterer Gegenstand der vorliegenden Erfindung ist die Verwendung der Gemische bei der Herstellung von Waschmitteln.

Unter dem Begriff "Haushaltsreiniger" oder "Reiniger" werden im Zusammenhang mit der vorliegenden Erfindung generell Formulierungen verstanden, die zum Reinigen von harten Oberflächen dienen. Sie liegen flüssig, als Gel, Paste oder auch fest vor. Zu den in fester Form gehörenden Materialien gehören Pulver und Kompaktate, wie beispielsweise Granulate und Formkörper, etwa Tabletten. Beispiele umfassen Handgeschirrspülmittel, maschinelle Geschirrreiniger, Metallentfetter, Glasreiniger, Fußbodenreiniger, Allzweckreiniger, Hochdruckreiniger, alkalische Reiniger, saure Reiniger, Spritzentfetter, Molkereireiniger, Polster-, Plastik-, und Badreiniger. Sie enthalten 0,01 bis 40 Gew.-%, bevorzugt 0,1 bis 25 Gew.-% bezogen auf die Gesamtformulierung mindestens einer Substanz der Formeln I und/oder II. Weitere Bestandteile sind nachfolgend aufgeführt.
- ionische Tenside, wie z. B. Alkoholsulfat/-ethersulfaten, Alkylbenzolsulfonate, α-Olefinsulfonate, Sulfosuccinate, wie oben unter "Waschmittel" beschrieben.
- nichtionische Tenside, wie z. B. Alkoholalkoxilate, Alkylaminalkoxilate, Alkylamidethoxilate, Alkylpolyglucoside, wie oben unter "Waschmittel" beschrieben.
- amphotere Tenside, wie z. B. Alkylaminoxide, Betaine, wie oben unter "Waschmittel" beschrieben.
- Builder wie z. B. Polyphosphate, Polycarboxilate, Phosphonate, Komplexbildner, z. B. Methylglycindiessigsäure und deren Salze, Nitrilotriessigsäure und deren Salze, Ethylendiamintetraessigsäure und deren Salze, wie oben unter "Waschmittel" beschrieben.
- Dispergiermittel, wie z. B. Naphthalinsulfonsäurekondensate, Polycarboxilate, wie oben unter "Waschmittel" beschrieben.
- pH-regulierende Verbindungen wie z. B. Alkalien (NaOH, KOH, Pentanatriummetasilikat) oder Säuren (Salzsäure, Phosphorsäure, Amidoschwefelsäure, Citronensäure)
- Enzyme, wie z. B. Lipasen, Amylasen, Proteasen
- Parfüm
- Farbstoffe
- Biozide, wie z. B. Isothiazolinone, 2-Bromo-2-nitro-1,3-propandiol, wie oben unter "Waschmittel" beschrieben.
- Bleichsysteme, bestehend aus Bleichmitteln, wie z. B. Perborat, Percarbonat etc., plus Bleichaktivatoren, wie z. B. Tetraacetylethylendiamin, plus Bleichstabilisatoren, wie oben unter "Waschmittel" beschrieben.
- Solubilisatoren, wie z. B. Cumolsulfonate, Toluolsulfonate, kurzkettige Fettsäuren, Phosphorsäurealkyl/-arylester
- Lösemittel, wie z. B. kurzkettige Alkyloligoglykole wie Butylglykol, Butyldiglykol, Propylenglykolmonomethylether, Alkohole wie Ethanol, i-Propanol, aromatische Lösemittel wie Toluol, Xylol, N-Alkylpyrrolidone, Alkylencarbonate

Die Bestandteile von Reinigern für harte Oberflächen sind dem Fachmann prinzipiell bekannt. Die obige Liste stellt nur einen exemplarischen Ausschnitt der Bestandteile dar.

Die Reiniger für harte Oberflächen sind gewöhnlich, aber nicht ausschließlich, wässrig und liegen in der Form von Mikroemulsionen, Emulsionen oder Lösungen vor.

Sollten sie in fester, pulverförmiger Form vorliegen, können zusätzlich Stellmittel wie z. B. Natriumsulfat, Magnesiumsulfat etc. eingesetzt werden.

Bei tablettenförmigen Reinigern werden zusätzlich Tablettierhilfsmittel wie z. B. Polyethylenglykole mit Molmassen > 1000 g/mol, Polymerdispersionen etc., und Tablettensprengmittel wie z. B. Cellulosederivate, vernetztes Polyvinylpyrrolidon, vernetzte Polyacrylate oder Kombinationen aus Säuren, z. B. Citronensäure plus Natriumbicarbonat, um nur einige zu nennen, benötigt.

In einer besonders bevorzugten Ausführungsform der vorliegenden Anmeldung handelt es sich bei den Reinigern um Handgeschirrspülmittel. Ein weiterer Gegenstand der vorliegenden Anmeldung ist daher ein Handgeschirrspülmittel enthaltend mindestens ein Alkylglycidolcarbonat der allgemeinen Formel I als Co-Tensid, sowie die Verwendung der Alkylglycidolcarbonate der allgemeinen Formel I als Co-Tenside in Handgeschirrspülmitteln.

Produkte aus dem Body-Care-Sektor sind beispielsweise Shampoos, Dusch- und Badegels, Dusch- und Badelotionen, Lippenstifte und kosmetische Formulierungen mit Pflege- und/oder Konditioniereigenschaften wie Stylingprodukte. Beispiele sind Haarschäume, Haargele, Haarsprays oder Nachbehandlungsmittel wie Haarwasser, Lotionen, Kurspülungen, Kurpackungen, Spitzenfluids, Hair-Repair-Mittel, "Hot Oil Treatments", Shampoos, Flüssigseifen, Pflegecremes, Haarfestiger, Haarfärbemittel und Dauerwellmittel. Bei Einsatz in Body-Care-Produkten weisen die Substanzen der Formel I den Vorteil auf, dass die physiologische Reizwirkung der Tensidmischungen abgemildert wird und die Schleimhäute geschützt werden.

Die Erfindung wird nun in den nachfolgenden Beispielen erläutert.

### Beispiel 1: Herstellung von 2-Propylheptylglycidolcarbonat

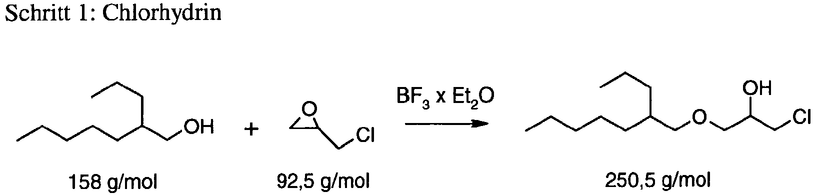

316 g (2 mol) 2-Propylheptanol wurden bei Raumtemperatur zusammen mit 1 g BF3-Diethyletheratkomplex vorgelegt. Man erwärmte auf 50 °C und dosierte dazu innerhalb von vier Stunden 186 g (2 mol) Epichlorhydrin. Die Mischung wurde noch 30 min bei 50 °C nachgerührt und anschließend auf Raumtemperatur abkühlen lassen. Nach Analyse (GC/MS) war das erwartete Produkt das Hauptprodukt (ca. 60%) der Synthese. Eine Aufreinigung durch Destillation ist möglich.

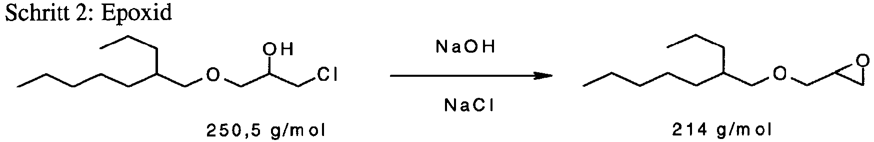

Zu 125.3 g (0.5 mol) des Chlorhydrins tropfte man bei Zimmertemperatur vorsichtig 159 g (1.0 mol) 25% NaOH (in Wasser). Dabei erwärmte man langsam auf 50 °C. Nach beendeter Zugabe erwärmte man weiter auf 100 °C und rührte bei dieser Temperatur für 15 Stunden. Nach dem Abkühlen auf Raumtemperatur trennte man die beiden Phasen. Die obere enthielt das gewünschte Produkt, welches durch Destillation gereinigt werden konnte. Ausbeute: 99%.

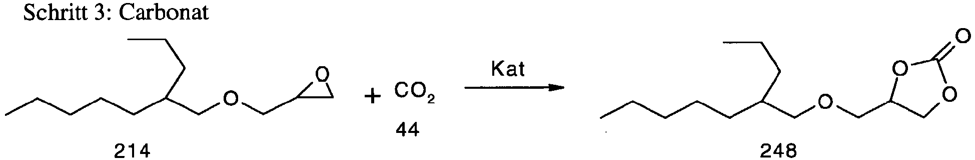

Das Epoxid (40 g, 0.15 mol) wurde zusammen mit einem Katalysator (0.42 g) in Aceton bei Zimmertemperatur vorgelegt. Katalysatoren für die Carbonat-Bildung sind z. B. beschrieben in: Paddock, Nguyen, J. Am. Chem. Soc. 2001, 123, 11498; Kisch, Millini, Wang, Chem. Ber. 1986, 119 (3), 1090; Baba, Nozaki, Matsuda, Bull. Chem. Soc. Jpn. 1987, 60 (4), 1552; Lermontov, Velikokhat'ko, Zavorin, Russ. Chem. Bull. 1998, 47 (7), 1405; Rokicki, Kuran, Pogorzelska-Marciniak, Monatshefte für Chemie 1984, 115, 205. Die Mischung wurde in einem Druckautoklaven auf 110 °C erhitzt und 14 bar CO₂ Druck aufgepresst. Dieser Druck wurde 11 Stunden gehalten, anschließend auf 50 °C abkühlen lassen und entspannt. Alle flüchtigen Anteile wurden am Rotationsverdampfer abgetrennt und das gewünschte Produkt als Sumpf der Destillation erhalten.

### Beispiel 2

### Handgeschirrspülmittel

Eine Modellformulierung enthaltend 30 Gew.-% Lutensit® ALBN50 (BASF AG, Alkylbenzolsulfonat, 50%ig), 10 Gew.-% Lutensol® AO7 (BASF AG, C13/15, Alkoholethoxylat, 7Ethylenoxid, 100%ig), 3 Gew.-% 2-Propylheptylglycidolcarbonat wird mit verschiedenen Mengen Lutensol^{®} A3N (BASF AG, C12,14-Alkoholethoxylat, 3EO, 100%ig BASF AG) versetzt. Die entstehenden Mischungen werden mit einem Uhbelohde-Viskosimeter, Spindel 3, Scherrate 3 s-1 untersucht. In Parallelexperimenten wurde eine entsprechende Tensidmischung, in der das Umsetzungsprodukt gegen Mazox®LDA (Laurylaminoxid, 100%ig, Herkunft BASF Corporation) und gegen Wasser ausgetauscht wurde, untersucht. Die Ergebnisse sind in der Tabelle zusammengefasst. Die Viskositätserhöhung ist bei dem erfindungsgemäßen Produkt am ausgeprägtesten.

| 0 | 1 | 2 | 4 | 6 | 8 | % Lutensol^{®} A3N |
|---|---|---|---|---|---|---|
| 3040 | 3440 | 8200 | 12300 | 18000 | 52000 | 2-Propylheptylglycidolcarbonat |
| 1210 | 905 | 970 | 1820 | 2890 | 7010 | Wasser |
| 2040 | 2500 | 2910 | 5760 | 12700 | 19200 | Mazox LDA Oxide w.S. |

### Beispiel 3

### Handgeschirrspülmittel

### Schaumstabilisierung mit 2-Propylheptylglycidolcarbonat

Eine Modellformulierung enthaltend 30 Gew.-% Lutensit® ALBN50 (Alkylbenzolsulfonat, 50%ig), 10 Gew.-% Lutensol® AO7 (C13/15, Alkoholethoxylat, 7Ethylenoxid, 100%ig), 3 Gew.-% 2-Propylheptylglycidolcarbonat und 3 Gew.-% Lutensol A3N (C12,14-Alkoholethoxylat, 3EO, 100%ig) wird auf 2 Gew.-% Tensid verdünnt. In einem Becherglas (5 1 Volumen, auf 2 1 gefüllt) wird diese Tensidlösung durch Rühren zum Schäumen versetzt. Wenn sich ein stabiler Zustand eingestellt hat, wird so lange frisches Olivenöl zugetropft, bis der Schaum verschwunden ist. Die dafür notwendige Menge Öl ist ein Maß für die Stabilität des Schaums. In Parallelexperimenten wurden eine entsprechende Tensidmischung, in der das Umsetzungsprodukt gegen Mazox®LDA (Laurylaminoxid, 100%ig) und gegen Wasser ausgetauscht wurde, untersucht. Die Ergebnisse sind in der Tabelle zusammengefasst.

| Zusatz | Verbrauch Olivenöl |
|---|---|
| Propylheptylglycidolcarbonat | 46 ml |
| Mazox®LDA | 28 ml |
| Wasser | 27 ml |

## Patentansprüche

1. Alkylglycidolcarbonate der allgemeinen Formel I 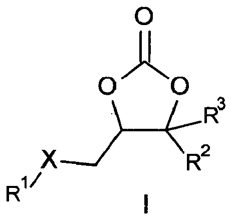 worin die Symbole X, R¹, R² und R³ die folgende Bedeutung aufweisen:
R¹ ist eine lineare oder verzweigte, unsubstituierte C₃-C₂₉-Alkylgruppe oder eine lineare oder verzweigte, unsubstituierte C₃-C₂₉-Alkenylgruppe, wobei der Substituent R¹ einen mittleren Verzweigungsgrad, der definiert ist als (Anzahl der Methylgruppen pro Molekül)-1, von 0,2 bis 1,6 aufweist;
R² und R³ sind unabhängig voneinander Wasserstoff oder eine lineare oder verzweigte Alkylgruppe;
X ist ausgewählt aus der Gruppe bestehend aus O und O(CH₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel I umfasst sind, worin n unterschiedliche Zahlenwerte aufweist,
wobei Alkylglycidolcarbonate der Formel 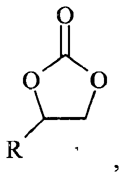 mit R = CH₂-O-CH(CH₃)₂ und mit R = CH₂-O-CH₂CH(CH₃)₂ ausgenommen sind.

2. Verbindungen nach Anspruch 1, **dadurch gekennzeichnet, dass** in der allgemeinen Formel 1 die Symbole X, R¹, R² und R³ die folgende Bedeutung aufweisen:
R¹ ist eine lineare oder verzweigte C₃-C₁₈-Alkylgruppe oder eine lineare oder verzweigte C₃-C₁₈-Alkenylgruppe, wobei der Substituent R¹ einen mittleren Verzweigungsgrad von 0,2 bis 1,6 aufweist;
R² und R³ sind unabhängig voneinander Wasserstoff oder eine lineare oder verzweigte Alkylgruppe mit 1 bis 5 Kohlenstoffatomen; und
X ist ausgewählt aus der Gruppe bestehend aus O und O(CH₂CHR⁴O) worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel I umfasst sind, worin n unterschiedliche Zahlenwerte aufweisen kann.

3. Verbindungen nach Anspruch 1 oder 2, **dadurch gekennzeichnet, dass** in der allgemeinen Formel I
R¹ eine lineare oder verzweigte C₅-C₁₈-Alkylgruppe oder eine lineare oder verzweigte C₅-C₁₈-Alkenylgruppe ist, wobei der Substituent einen mittleren Verzweigungsgrad von 0,2 bis 1,6 aufweist; und
mindestens einer der Reste R² oder R³ Wasserstoff ist.

4. Verbindungen nach einem der Ansprüche 1 bis 3, **dadurch gekennzeichnet, dass** R² und R³ Wasserstoff bedeuten.

5. Verbindungen nach Anspruch 4, **dadurch gekennzeichnet, dass** in der allgemeinen Formel I R¹-X C₅H₁₁CH(C₃H₇)CH₂O bedeutet, oder ein auf einem technischen C13-C15-Oxoalkohol oder einem technischen oder nativen C12-C14-Alkohol oder einem C10- oder C13-Alkohol basierender Rest mit einem Verzweigungsgrad von ca. 1,5 ist.

6. Verbindungen nach Anspruch 5, **dadurch gekennzeichnet, dass** R¹-X C₅H₁₁CH(C₃H₇)CH₂O bedeutet, und dass sie als Gemisch vorliegen in dem 70 bis 99 Gew% Verbindungen enthalten sind, bei denen C₅H₁₁ die Bedeutung n-C₅H₁₁ hat und
1 bis 30 Gew% Verbindungen enthalten sind, bei denen C₅H₁₁ die Bedeutung C₂H₅CH(CH₃)CH₂ und/oder CH₃CH(CH₃)CH₂CH₂ hat.

7. Alkylglycidolcarbonate der allgemeinen Formel I 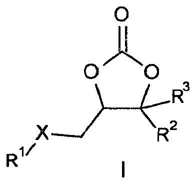 worin die Symbole X, R¹, R² und R³ die folgende Bedeutung aufweisen:
R¹ ist eine lineare oder verzweigte, unsubstituierte C₃-C₂₉-Alkylgruppe oder eine lineare oder verzweigte, unsubstituierte C₃-C₂₉-Alkenylgruppe;
R² und R³ sind unabhängig voneinander Wasserstoff oder eine lineare oder verzweigte Alkylgruppe;
X ist O(CH₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel I umfasst sind, worin n unterschiedliche Zahlenwerte aufweist.

8. Verbindungen nach Anspruch 7, **dadurch gekennzeichnet, dass** der Substituent R¹ einen mittleren Verzweigungsgrad, der definiert ist als (Anzahl der Methylgruppen pro Molekül)-1, von 0 bis 2,5 aufweist.

9. Verfahren zur Herstellung von Alkylglycidolcarbonaten nach einem der Ansprüche 1 bis 8, durch Umsetzung von mit einer R¹-X-CH₂-Gruppe funktionalisierten 1,2-Diolen der allgemeinen Formel II mit Phosgen gemäß dem folgenden Reaktionsschema: 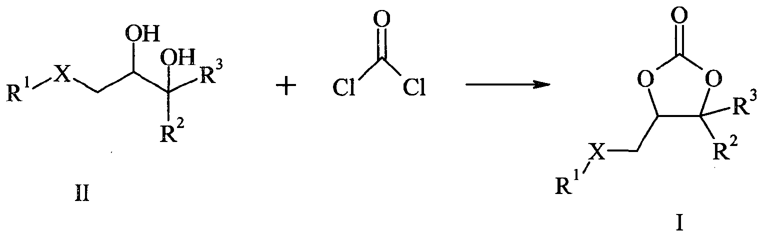 worin die Symbole X, R¹, R² und R³ die folgende Bedeutung aufweisen:
R¹ ist eine lineare oder verzweigte, unsubstituerte C₃-C₂₉-Alkylgruppe oder eine lineare oder verzweigte, unsubstituierte C₃-C₂₉-Alkenylgruppe;
R² und R³ sind unabhängig voneinander Wasserstoff oder eine lineare oder verzweigte Alkylgruppe;
X ist ausgewählt aus der Gruppe bestehend aus O und O(CH₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel I umfasst sind, worin n unterschiedliche Zahlenwerte aufweist.

10. Verfahren zur Herstellung von Alkylglycidolcarbonaten gemäß einem der Ansprüche 1 bis 8 durch Umsetzung von Epoxiden der Formel IV gemäß dem folgenden Reaktionsschema mit CO₂ unter Einsatz eines Katalysators: 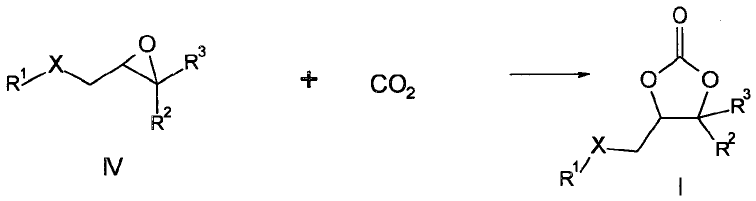 worin die Symbole X, R¹, R² und R³ die folgende Bedeutung aufweisen:
R¹ ist eine lineare oder verzweigte, unsubstituierte C₃-C₂₉-Alkylgruppe oder eine lineare oder verzweigte, unsubstituierte C₃-C₂₉-Alkenylgruppe;
R² und R³ sind unabhängig voneinander Wasserstoff oder eine lineare oder verzweigte Alkylgruppe;
X ist ausgewählt aus der Gruppe bestehend aus O und O(CH₂CHR⁴O)ₙ, worin R⁴ Wasserstoff, Methyl, Ethyl oder Propyl bedeutet und n eine Zahl von 1 bis 5 ist, wobei auch Mischungen von Verbindungen mit Gruppen X der Formel O(CH₂CHR⁴O)ₙ von der allgemeinen Formel I umfasst sind, worin n unterschiedliche Zahlenwerte aufweist.

11. Verfahren nach Anspruch 10, **dadurch gekennzeichnet, dass** das Epoxid der Formel IV durch Umsetzung von Epichlorhydrin mit geeigneten Alkoholen oder mit Alkylenoxiden umgesetzten Alkoholen, und anschließender oder gleichzeitiger Eliminierung von HCl hergestellt wird.

12. Verfahren nach Anspruch 11, **dadurch gekennzeichnet, dass** die geeigneten Alkohole oder mit Alkylenoxiden umgesetzten Alkohole ausgewählt sind aus linearen oder verzweigten aliphatischen C₃-C₂₉-Alkoholen mit einem mittleren Verzweigungsgrad, der definiert ist als (Anzahl der Methylgruppen pro Molekül)-1, von 0 bis 2,5, wobei die Alkylkette weitere Substituenten aufweisen kann, die die Eignung des Moleküls als Co-Tensid erhöhen, diese aber zumindest nicht negativ beeinflussen, Guerbet-Alkoholen und ihren ungesättigten Analoga, und den entsprechenden geeigneten mit Alkylenoxiden umgesetzten Alkoholen.

13. Verwendung von Alkylglycidolcarbonaten gemäß einem der Ansprüche 1 bis 8 oder eines Gemischs davon als Co-Tensid.

14. Haushaltswaschmittel, Haushaltsreiniger, Körperreinigungsmittel oder Körperpflegemittel enthaltend mindestens ein Alkylglycidolcarbonat gemäß einem der Ansprüche 1 bis 8.

15. Waschmittel nach Anspruch 14 in fester, flüssiger, gelförmiger oder pastenförmiger Form, vorzugsweise als Pulver, Kompaktat, Granulat, Tablette oder Gel.

16. Waschmittel nach Anspruch 14 oder 15 enthaltend 0,1 bis 40 Gew-%, bezogen auf die Gesamtmenge der Formulierung, mindestens eines Alkylglycidolcarbonats gemäß einem der Ansprüche 1 bis 8.

17. Haushaltsreiniger nach Anspruch 14 in flüssiger, gelförmiger oder fester Form, vorzugsweise als Flüssigkeit, Gel, Pulver oder Kompaktat.

18. Haushaltsreiniger nach Anspruch 17 in Form eines Handgeschirrspülmittels, maschinellen Geschirrreinigers, Metallentfetters, Glasreinigers, Fußbodenreinigers, Allzweckreinigers, Hochdruckreinigers, alkalische Reinigers, sauren Reinigers, Spritzentfetters, Molkereireinigers, Polster-, Plastik-, und Badreinigers.

19. Haushaltsreiniger nach Anspruch 17 oder 18 enthaltend 0,01 bis 40 Gew.-%, bezogen auf die Gesamtformulierung, mindestens eines Alkylglycidolcarbonats gemäß einem der Ansprüche 1 bis 8.

20. Körperreinigungsmittel oder Körperpflegemittel in Form eines Shampoos, Dusch- oder Badegels, Dusch- oder Badelotion, eines Lippenstifts, einer kosmetischen Formulierungen mit Pflege- und/oder Konditioniereigenschaften oder eines Stylingprodukts, insbesondere einer Flüssigseife, einer Pflegecreme, eines Haarschaums, Haargels, Haarsprays oder Nachbehandlungsmittels, eines Haarwassers, einer Lotion, Kurspülung, Kurpackung, eines Spitzenfluids, Hair-Repair-Mittels, "Hot Oil Treatments", Haarfestigers, Haarfärbemittels oder Dauerwellmittels, enthaltend mindestens ein Alkyglycidolcarbonat gemäß einem der Ansprüche 1 bis 8.

## Claims

1. An alkylglycidol carbonate of the formula I 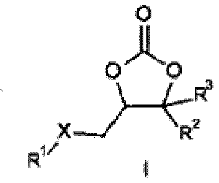 in which the symbols X, R¹, R² and R³ have the following meanings:
R¹ is a linear or branched, unsubstituted C₃-C₂₉-alkyl group or a linear or branched, unsubstituted C₃-C₂₉-alkenyl group, where the substituent R¹ has an average degree of branching, which is defined as (number of methyl groups per molecule)-1, of from 0.2 to 1.6;
R² and R³, independently of one another, are hydrogen or a linear or branched alkyl group;
X is chosen from the group consisting of O and O(CH₂CHR⁴O)ₙ, in which R⁴ is hydrogen, methyl, ethyl or propyl, and n is a number from 1 to 5, where mixtures of compounds with groups X of the formula O(CH₂CHR⁴O)ₙ are also included by the formula I, in which n has various numerical values,
where alkylglycidol carbonates of the formula 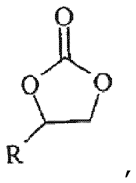 where R = CH₂-O-CH(CH₃)₂ and where R = CH₂-O-CH₂CH(CH₃)₂
are excluded.

2. A compound according to claim 1, wherein in the formula I the symbols X, R¹, R² and R³ have the following meanings:
R¹ is a linear or branched C₃-C₁₈-alkyl group or a linear or branched C₃-C₁₈-alkenyl group, where the substituent R¹ has an average degree of branching of from 0.2 to 1.6;
R² and R³, independently of one another, are hydrogen or a linear or branched alkyl group having 1 to 5 carbon atoms; and
X is chosen from the group consisting of O and O(CH₂CHR⁴O)ₙ, in which R⁴ is hydrogen, methyl, ethyl or propyl and n is a number from 1 to 5, where mixtures of compounds with groups X of the formula O(CH₂CHR⁴O)ₙ are covered by the formula I, in which n can have various numerical values.

3. A compound according to claim 1 or 2, wherein in the formula I
R¹ is a linear or branched C₅-C₁₈-alkyl group or a linear or branched C₅-C₁₈-alkenyl group, where the substituent has an average degree of branching of from 0.2 to 1.6; and
at least one of the radicals R² or R³ is hydrogen.

4. A compound according to any of claims 1 to 3, wherein R² and R³ are hydrogen.

5. A compound according to claim 4, wherein in the formula I R¹-X is C₅H₁₁CH(C₃H₇)CH₂O, or a radical based on a technical-grade C₁₃-C₁₅-oxo alcohol or a technical-grade or native C₁₂-C₁₄-alcohol or a C₁₀- or C₁₃-alcohol and having a degree of branching of about 1.5.

6. A compound according to claim 5, wherein R¹-X is C₅H₁₁CH(C₃H₇)CH₂O, and wherein it is present as a mixture in which
70 to 99% by weight of compounds in which C₅H₁₁ has the meaning n-C₅H₁₁ are present and
1 to 30% by weight of compounds in which C₅H₁₁ has the meaning C₂H₅CH(CH₃)CH₂ and/or CH₃CH(CH₃)CH₂CH₂ are present.

7. An alkylglycidol carbonate of the formula I 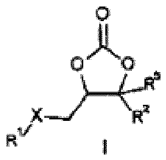 in which the symbols X, R¹, R² and R³ have the following meanings:
R¹ is a linear or branched, unsubstituted C₃-C₂₉-alkyl group or a linear or branched, unsubstituted C₃-C₂₉-alkenyl group;
R² and R³, independently of one another, are hydrogen or a linear or branched alkyl group;
X is O(CH₂CHR⁴O)ₙ, in which R⁴ is hydrogen, methyl, ethyl or propyl, and n is a number from 1 to 5, where mixtures of compounds with groups X of the formula O(CH₂CHR⁴O)ₙ are also included by the formula I, in which n has various numerical values.

8. A compound according to claim 7, wherein the substituent R¹ has an average degree of branching, which is defined as (number of methyl groups per molecule)-1, of from 0 to 2.5.

9. A method for producing alkylglycidol carbonates according to any of claims 1 to 8, by reacting 1,2-diols of the formula II and functionalized with an R¹-X-CH₂ group with phosgene in accordance with the following reaction scheme: 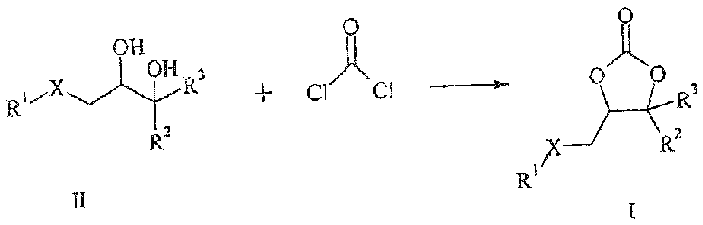 in which the symbols X, R¹, R² and R³ have the following meanings:
R¹ is a linear or branched, unsubstituted C₃-C₂₉-alkyl group or a linear or branched, unsubstituted C₃-C₂₉-alkenyl group;
R² and R³, independently of one another, are hydrogen or a linear or branched alkyl group;
X is chosen from the group consisting of O and O(CH₂CHR⁴O)ₙ, in which R⁴ is hydrogen, methyl, ethyl or propyl, and n is a number from 1 to 5, where mixtures of compounds with groups X of the formula O(CH₂CHR⁴O)ₙ are also included by the formula I, in which n has various numerical values.

10. A method for producing alkylglycidol carbonates according to any of claims 1 to 8 by reacting epoxides of the formula IV according to the following reaction scheme with CO₂ using a catalyst: 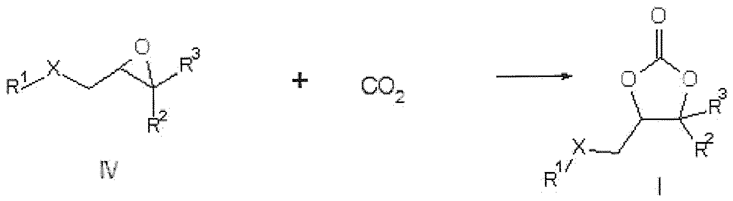 in which the symbols X, R¹, R² and R³ have the following meanings:
R¹ is a linear or branched, unsubstituted C₃-C₂₉-alkyl group or a linear or branched, unsubstituted C₃-C₂₉-alkenyl group;
R² and R³, independently of one another, are hydrogen or a linear or branched alkyl group;
X is chosen from the group consisting of O and O(CH₂CHR⁴O)ₙ, in which R⁴ is hydrogen, methyl, ethyl or propyl, and n is a number from 1 to 5, where mixtures of compounds with groups X of the formula O(CH₂CHR⁴O)ₙ are also included by the formula I, in which n has various numerical values.

11. The method according to claim 10, wherein the epoxide of the formula IV is produced by reacting epichlorohydrin with suitable alcohols or alcohols reacted with alkylene oxides, and subsequent or simultaneous elimination of HCl.

12. The method according to claim 11, wherein the suitable alcohols or alcohols reacted with alkylene oxides are chosen from linear or branched aliphatic C₃-C₂₉-alcohols with an average degree of branching, which is defined as (number of methyl groups per molecule)-1, of from 0 to 2.5, where the alkyl chain can have further substituents which increase the suitability of the molecule as cosurfactant, but at least do not negatively influence it, Guerbet alcohols and their unsaturated analogs, and the corresponding suitable alcohols reacted with alkylene oxides.

13. The use of alkylglycidol carbonates according to any of claims 1 to 8 or a mixture thereof as cosurfactant.

14. A household detergent, household cleaner, body-cleansing composition or bodycare composition comprising at least one alkylglycidol carbonate according to any of claims 1 to 8.

15. A detergent according to claim 14 in solid, liquid, gel or paste form, preferably in the form of a powder, compact, granules, tablet or gel.

16. A detergent according to claim 14 or 15, comprising 0.1 to 40% by weight, based on the total amount of the formulation, of at least one alkylglycidol carbonate according to any of claims 1 to 8.

17. A household cleaner according to claim 14 in liquid, gel or solid form, preferably in the form of a liquid, gel, powder or compact.

18. A household cleaner according to claim 17 in the form of a hand dishwashing detergent, machine dishwashing detergent, metal degreaser, glass cleaner, floor cleaner, all-purpose cleaner, highpressure cleaner, alkaline cleaner, acidic cleaner, spray degreaser, dairy cleaner, upholstery cleaner, plastic cleaner and bathroom cleaner.

19. A household cleaner according to claim 17 or 18, comprising 0.01 to 40% by weight, based on the total formulation, of at least one alkylglycidol carbonate according to any of claims 1 to 8.

20. A body-cleansing composition or bodycare composition in the form of a shampoo, shower or bath gel, shower or bath lotion, a lipstick, a cosmetic formulation with care and/or conditioning properties or a styling product, in particular a liquid soap, a care cream, a hair foam, hair gel, hair spray or after-treatment composition, a hair tonic, a lotion, treatment rinse, treatment pack, a split-end fluid, hair repair composition, hot oil treatment, hair-setting composition, hair colorant or permanent waving agent, comprising at least one alkylglycidol carbonate according to any of claims 1 to 8.

## Revendications

1. Carbonates d'alkylglycidol de formule générale I 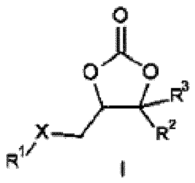 dans laquelle les symboles X, R¹, R² et R³ ont la signification suivants :
R¹ est un groupe alkyle en C₃-C₂₉ linéaire ou ramifié, non substitué ou un groupe alcényle en C₃-C₂₉ linéaire ou ramifié, non substitué, le substituant R¹ présentant un degré de ramification moyen, qui est défini comme (nombre des groupes méthyle par molécule) - 1, de 0,2 à 1, 6 ;
R² et R³ représentent, indépendamment l'un de l'autre, un atome d'hydrogène ou un groupe alkyle linéaire ou ramifié ;
X est choisi dans le groupe constitué par O et O(CH₂CHR⁴O)ₙ, où R⁴ représente un atome d'hydrogène, le groupe méthyle, éthyle ou propyle et n est un nombre valant de 1 à 5, des mélanges de composés à groupes X de formule O(CH₂CHR⁴O)ₙ, dans laquelle n présente différentes valeurs numériques, étant également englobés par la formule générale I,
les carbonates d'alkylglycidol de formule 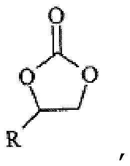 où R = CH₂-O-CH(CH₃)₂ et où R = CH₂-O-CH₂CH(CH₃)₂ étant exclus.

2. Composés selon la revendication 1, **caractérisés en ce que** dans la formule générale I les symboles X, R¹, R² et R ont la signification suivante :
R¹ est un groupe alkyle en C₃-C₁₈ linéaire ou ramifié ou un groupe alcényle en C₃-C₁₈ linéaire ou ramifié, le substituant R¹ présentant un degré de ramification moyen de 0,2 à 1,6 ;
R² et R³ représentent, indépendamment l'un de l'autre, un atome d'hydrogène ou un groupe alkyle linéaire ou
ramifié ayant de 1 à 5 atomes de carbone ; et X est choisi dans le groupe constitué par O et O(CH₂CHR⁴O)ₙ, où R⁴ représente un atome d'hydrogène, le groupe méthyle, éthyle ou propyle et n est un nombre valant de 1 à 5, des mélanges de composés à groupes X de formule O(CH₂CHR⁴O)ₙ, dans laquelle n peut présenter différentes valeurs numériques, étant également englobés par la formule générale I.

3. Composés selon la revendication 1 ou 2, **caractérisés en ce que** dans la formule générale I
R¹ est un groupe alkyle en C₅-C₁₈ linéaire ou ramifié ou un groupe alcényle en C₅-C₁₈ linéaire ou ramifié, le substituant présentant un degré de ramification moyen de 0,2 à 1,6 ; et
au moins l'un des radicaux R² ou R³ est un atome d'hydrogène.

4. Composés selon l'une quelconque des revendications 1 à 3, **caractérisés en ce que** R² et R³ représentent des atomes d'hydrogène.

5. Composés selon la revendication 4, **caractérisés en ce que** dans la formule générale I R¹-X représente C₅H₁₁CH(C₃H₇)CH₂O, ou est un radical ayant un degré de ramification d'environ 1,5, se fondant sur un oxoalcool en C₁₃-C₁₅ industriel ou sur un alcool en C₁₂-C₁₄ naturel ou industriel ou sur un alcool en C₁₀ ou C₁₃.

6. Composés selon la revendication 5, **caractérisés en ce que** R¹-X représente C₅H₁₁CH(C₃H₇)CH₂O, et **en ce qu'**ils se trouvent sous forme de mélange dans lequel sont contenus 70 à 99 % en poids de composés dans lesquels C₅H₁₁ a la signification de n-C₅H₁₁ et
sont contenus 1 à 30 % en poids de composés dans lesquels C₅H₁₁ a la signification de C₂H₅CH(CH₃)CH₂ et/ou CH₃CH(CH₃)CH₂CH₂.

7. Carbonates d'alkylglycidol de formule générale I 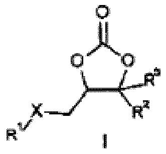 dans laquelle les symboles X, R¹, R et R ont les significations suivantes :
R¹ est un groupe alkyle en C₃-C₂₉ linéaire ou ramifié, non substitué ou un groupe alcényle en C₃-C₂₉ linéaire ou ramifié, non substitué ;
R² et R³ représentent, indépendamment l'un de l'autre, un atome d'hydrogène ou un groupe alkyle linéaire ou ramifié ;
X est O(CH₂CHR⁴O)ₙ, où R⁴ représente un atome d'hydrogène, le groupe méthyle, éthyle ou propyle et n est un nombre valant de 1 à 5, des mélanges de composés à groupes X de formule O(CH₂CHR⁴O)ₙ, dans laquelle n présente différentes valeurs numériques, étant également englobés par la formule générale I.

8. Composés selon la revendication 7, **caractérisés en ce que** le substituant R¹ présentant un degré de ramification moyen, qui est défini comme (nombre des groupes méthyle par molécule) - 1, de 0 à 2,5.

9. Procédé pour la préparation de carbonates d'alkylglycidol selon l'une quelconque des revendications 1 à 8, par mise en réaction de 1,2-diols de formule générale II, fonctionnalisés avec un groupe R¹-X-CH₂, avec du phosgène selon le schéma de réaction suivant : 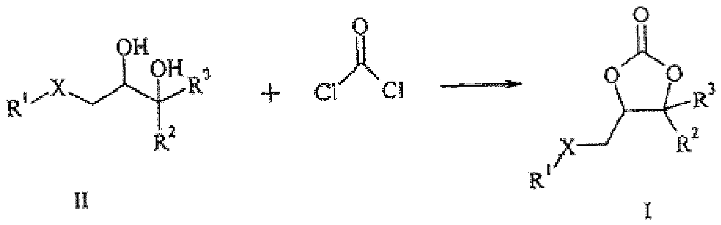 dans lequel les symboles X, R¹, R² et R³ ont les significations suivantes :
R¹ est un groupe alkyle en C₃-C₂₉ linéaire ou ramifié, non substitué ou un groupe alcényle en C₃-C₂₉ linéaire ou ramifié, non substitué ;
R² et R³ représentent, indépendamment l'un de l'autre, un atome d'hydrogène ou un groupe alkyle linéaire ou ramifié ;
X est choisi dans le groupe constitué par O et O(CH₂CHR⁴O)ₙ, où R⁴ représente un atome d'hydrogène, le groupe méthyle, éthyle ou propyle et n est un nombre valant de 1 à 5, des mélanges de composés à groupes X de formule O(CH₂CHR⁴O)ₙ, dans laquelle n présente différentes valeurs numériques, étant également englobés par la formule générale I.

10. Procédé pour la préparation de carbonates d'alkylglycidol selon l'une quelconque des revendications 1 à 8, par mise en réaction d'époxydes de formule IV avec CO₂ selon le schéma de réaction suivant, avec utilisation d'un catalyseur : 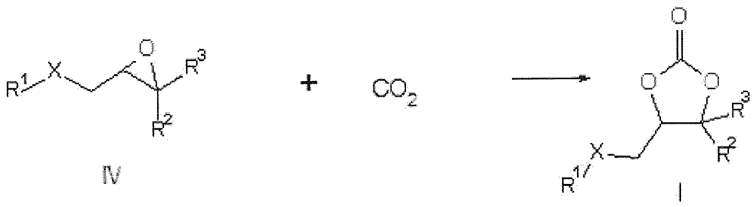 où les symboles X, R¹, R² et R³ ont la signification suivants :
R¹ est un groupe alkyle en C₃-C₂₉ linéaire ou ramifié, non substitué ou un groupe alcényle en C₃-C₂₉ linéaire ou ramifié, non substitué ;
R² et R³ représentent, indépendamment l'un de l'autre, un atome d'hydrogène ou un groupe alkyle linéaire ou ramifié ;
X est choisi dans le groupe constitué par O et O(CH₂CHR⁴O)ₙ, où R⁴ représente un atome d'hydrogène, le groupe méthyle, éthyle ou propyle et n est un nombre valant de 1 à 5, des mélanges de composés à groupes X de formule O(CH₂CHR⁴O)ₙ, dans laquelle n présente différentes valeurs numériques, étant également englobés par la formule générale I.

11. Procédé selon la revendication 10, **caractérisé en ce que** l'époxyde de formule IV est préparé par mise en réaction d'épichlorhydrine avec des alcools appropriés ou des alcools appropriés ayant réagi avec des oxydes d'alkylène, et élimination simultanée ou subséquente de HCl.

12. Procédé selon la revendication 11, **caractérisé en ce que** les alcools appropriés ou alcools appropriés ayant réagi avec des oxydes d'alkylène sont choisis parmi des alcools aliphatiques en C₃-C₂₉ linéaires ou ramifiés ayant un degré de ramification moyen, qui est défini comme (nombre des groupes méthyle par molécule) - 1, de 0 à 2,5, la chaîne alkyle pouvant comporter d'autres substituants qui augmentent la qualification de la molécule en tant que co-tensioactif, mais au moins n'ont pas d'effet négatif sur celle-ci, des alcools de Guerbet et leurs analogues insaturés, et des alcools appropriés correspondants ayant réagi avec des oxydes d'alkylène.

13. Utilisation de carbonates d'alkylglycidol selon l'une quelconque des revendications 1 à 8, ou d'un mélange de ceux-ci, en tant que co-tensioactif.

14. Produit de lavage ménager, produit de nettoyage ménager, produit pour le nettoyage du corps ou produit pour le soin du corps, contenant au moins un carbonate d'alkylglycidol selon l'une quelconque des revendications 1 à 8.

15. Produit de lavage selon la revendication 14, sous forme solide, liquide, de gel ou de pâte, de préférence sous forme de poudre, produit compacté, granulé, tablette ou gel.

16. Produit de lavage selon la revendication 14 ou 15, contenant de 0,1 à 40 % en poids, par rapport à la quantité totale de la composition, d'au moins un carbonate d'alkylglycidol selon l'une quelconque des revendications 1 à 8.

17. Produit de nettoyage ménager selon la revendication 14, sous forme liquide, de gel ou solide, de préférence sous forme de liquide, gel, poudre ou produit compacté.

18. Produit de nettoyage ménager selon la revendication 17, sous forme d'un produit pour lavage de la vaisselle à la main, produit pour lavage de la vaisselle en machine, dégraissant pour métaux, produit de nettoyage pour le verre, produit de nettoyage pour sols, produit de nettoyage tous usages, produit de nettoyage sous haute pression, produit de nettoyage alcalin, produit de nettoyage acide, produit pour le dégraissage au jet, produit de nettoyage pour laiteries, produit de nettoyage pour meubles rembourrés, matières plastiques et salles de bain.

19. Produit de nettoyage ménager selon la revendication 17 ou 18, contenant de 0,01 à 40 % en poids, par rapport à la composition totale, d'au moins un carbonate d'alkylglycidol selon l'une quelconque des revendications 1 à 8.

20. Produit pour le nettoyage du corps ou produit pour le soin du corps, sous forme d'un shampooing, d'un gel douche ou bain, d'une lotion pour la douche ou le bain, d'un bâton pour les lèvres, d'une composition cosmétique à propriétés de soin et/ou de conditionnement ou d'un produit de coiffage, en particulier d'un savon liquide, d'une crème de soin, d'une mousse capillaire, d'un gel capillaire, d'une laque pour cheveux ou d'un produit d'après-traitement, d'une lotion capillaire, d'une lotion, d'un après-shampooing traitant, d'un masque capillaire, d'un sérum anti-fourches, d'un produit Hair-Repair, de « Hot Oil Treatments », d'un fixateur pour cheveux, d'un produit colorant pour cheveux ou d'un produit pour ondulation permanente, contenant au moins un carbonate d'alkylglycidol selon l'une quelconque des revendications 1 à 8.
